(19) Europäisches Patentamt
European Patent Office
Office européen des brevets

(11) Veröffentlichungsnummer: **0 237 963**
**A2**

# (12) EUROPÄISCHE PATENTANMELDUNG

(21) Anmeldenummer: **87103589.5**

(22) Anmeldetag: **12.03.87**

(51) Int. Cl.[3]: **C 07 D 405/14**
C 07 D 409/14, C 07 D 213/04
C 07 D 215/02, C 07 D 237/30
C 07 D 239/00, A 61 K 31/495
A 61 K 31/435
//A61K31/41, A61K31/415

(30) Priorität: **21.03.86 DE 3609596**

(43) Veröffentlichungstag der Anmeldung:
**23.09.87 Patentblatt 87/39**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kampe, Klaus-Dieter, Dr.**
**Am Rehsteig 1**
**D-6232 Bad Soden am Taunus(DE)**

(72) Erfinder: **Raether, Wolfgang, Dr.**
**Falkensteinstrasse 6**
**D-6072 Dreieich(DE)**

(72) Erfinder: **Dittmar, Walter, Dr.**
**Uhlandstrasse 10**
**D-6238 Hofheim am Taunus(DE)**

(72) Erfinder: **Hänel, Heinz, Dr.**
**Tannenwaldallee 80**
**D-6380 Bad Homburg(DE)**

## (54) 2-Azolylmethyl-2-aryl-1,3-dioxolane und deren Salze, Verfahren zu ihrer Herstellung, sie enthaltende Mittel und ihre Verwendung.

(57) Beschrieben werden Verbindungen I

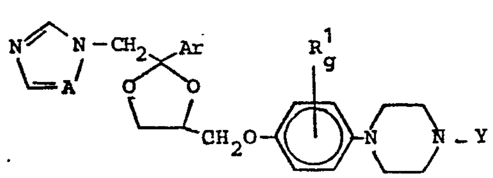

mit A gleich CH oder N; Ar gleich Naphtyl, Thienyl, Phenyl; $R^1$ gleich Alkyl, F, Cl; g gleich null, 1, 2; Y gleich verschiedene heterocyclische Basen sowie deren Säureadditionssalze.

Beschrieben werden mehrere Herstellungsverfahren.

Verbindungen IIIa

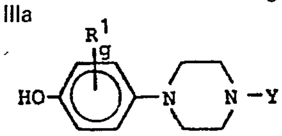

mit $R^1$, g und Y wie zu Formel I angegeben dienen als Zwischenprodukte zu deren Herstellung. Auch für die Herstellung von IIIa sind Verfahren angegeben.

I stellen wertvolle Antimykotika dar.

EP 0 237 963 A2

Croydon Printing Company Ltd.

2-Azolylmethyl-2-aryl-1,3-dioxolane und deren Salze, Verfahren zu ihrer Herstellung, sie enthaltende Mittel und ihre Verwendung

Die Erfindung betrifft mit Heterocyclen substituierte 2-Azolylmethyl-2-aryl-4-[(4-piperazino-phenoxy)-methyl]-1,3-dioxolane einschließlich ihrer Salze, Verfahren zu ihrer Herstellung, diese Verbindungen enthaltende Arzneimittel und ihre Verwendung insbesondere als Antimykotika.

Antimykotisch bzw. fungizid wirksame 2-Azolylmethyl-2-aryl-4-[(4-piperazino-phenoxy)-methyl]-1,3-dioxolane sind bereits bekannt und werden u.a. in der DE-OS 2 804 096, und der EP-OS 7696 beschrieben. Die bekanntesten Vertreter aus der großen Zahl der beschriebenen Verbindungen sind 2-S,(R)-(2,4-Dichlorphenyl)-2-(imidazol-1-ylmethyl)-4-R,(S)-[4-(4-acetyl-piperazin-1-yl)-phenoxymethyl]-1,3-dioxolan (Ketoconazol) und 2-S,(R)-(2,4-Dichlorphenyl)-2-(1,2,4-triazol-1-ylmethyl)-4-R,(S)-[4-(4-isopropyl-piperazin-1-yl)-phenoxymethyl]-1,3-dioxolan (Terconazol), die als Antimykotika im Handel sind (vgl. DOS 2 804 096, Beispiel 20 und Beispiel 53), wobei Ketoconazol vorwiegend als systemisch wirksames und Terconazol als topisch wirksames Antimykotikum verwendet wird. Die antimykotische Wirkung sowie insbesondere die Verträglichkeit der bekannten Verbindungen sind jedoch nicht immer ganz befriedigend.

Es wurde nun gefunden, daß 2-Azolylmethyl-2-aryl-4-[(4-piperazino-phenoxy)-methyl]-1,3-dioxolane der Formel

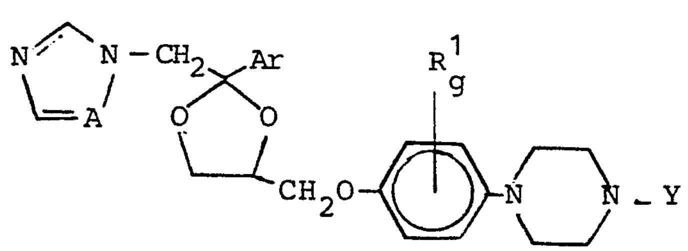

I,

in der

A CH oder N,

Ar Naphthyl, Thienyl, Halothienyl oder eine unsubstituierte oder eine bis zu 3 Substituenten tragende Phenylgruppe bedeutet, wobei die Substituenten gleich oder verschieden sein können und Halogen, Trifluormethyl, $C_1$-$C_2$-Alkyl, $C_1$-$C_2$-Alkoxy oder Phenoxy bedeuten,

$R^1$ $C_1$-$C_3$-Alkyl, F oder Cl ,

g 0, 1 oder 2,

Y die folgenden heterocyclischen Reste

a) 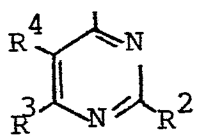 oder 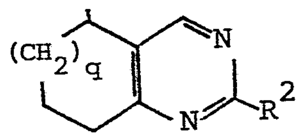 , in denen

$R^2$ $C_1$-$C_4$-Alkyl, eine unsubstituierte oder 1 oder 2 Substituenten tragende Phenylgruppe, wobei die Substituenten gleich oder verschieden sein können und Halogen, Trifluormethyl, Methoxy, Ethoxy, Nitro oder $C_1$-$C_4$-Alkyl bedeuten,
oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Methoxy-, Ethoxy- oder $C_1$-$C_3$-Alkyl bedeuten,

$R^3$ H, $C_1$-$C_8$-Alkyl, $C_3$-$C_6$-Cycloalkyl-$C_1$-$C_3$-alkyl, eine unsubstituierte oder 1 oder 2 Substituenten tragende Phenylgruppe, wobei die Substituenten gleich oder verschieden sein können und Halogen, Methoxy-, Ethoxy oder $C_1$-$C_3$-Alkyl bedeuten, eine unsubstituierte oder im Phenylrest durch Methoxy, 1,2-Methylendioxy, F, Cl oder $C_1$-$C_3$-Alkyl substituierte Phenyl-$C_1$-$C_2$-alkylgruppe, oder Trifluormethyl

$R^4$ H, $C_1$-$C_4$-Alkyl oder Benzyl,

oder $R^3$ und $R^4$ zusammen $-(CH_2)_r-$, wobei r=3 oder 4 ist, oder -CH=CH-CH=CH-,

q 0 oder 1 bedeutet, oder

b) 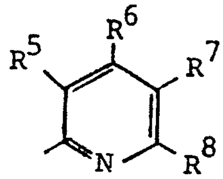 , in dem

$R^5$ H oder CN

$R^6$ H, $C_1$-$C_4$-Alkyl oder eine unsubstituierte oder durch $OCH_3$, F, Cl, $CH_3$ oder $C_2H_5$ substituierte Phenylgruppe

$R^7$ H, Benzyl, $CF_3$ oder $CH_3$,

$R^8$ $C_5$-$C_6$-Cycloalkyl oder eine unsubstituierte oder durch $OCH_3$, F, Cl, $CH_3$ oder $C_2H_5$ substituierte Phenylgruppe bedeutet und, falls $R^5$ CN und/oder $R^7$ $CF_3$ bedeutet, $R^8$ auch H bedeuten kann, oder

$R^7$ und $R^8$ zusammen $-(CH_2)_4-$ bedeuten, oder

c) 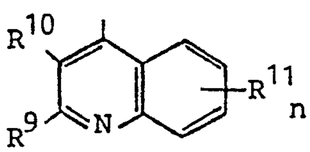 , in dem

$R^9$ H, Methyl oder Ethyl,

$R^{10}$ H, CN oder $COOR^{12}$, wobei $R^{12}$ Methyl oder Ethyl bedeutet,

$R^{11}$ $C_1$-$C_4$-Alkyl, $C_1$-$C_4$-Alkoxy, Halogen oder Trifluormethyl und

n 0, 1 oder 2 bedeutet, wobei, falls $R^{11}$ $CF_3$ bedeutet, n=1 ist, und, falls n$\neq$0, die Reste $R^{11}$ in 5, 6, 7 oder 8-Stellung des Chinolinsystems stehen können, oder

d) 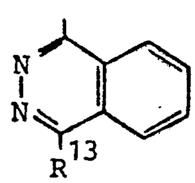 , in dem

$R^{13}$ H, $C_1$-$C_4$-Alkyl oder eine unsubstituierte oder 1 oder 2 Substituenten tragende Phenylgruppe, wobei die Substituenten gleich oder verschieden sein

können und Halogen, Methoxy, Ethoxy, Methyl oder Ethyl bedeuten, bedeutet,

sowie deren physiologisch verträgliche Säureadditionssalze wertvolle antimykotische bzw. fungizide Eigenschaften besitzen. Sie sind daher zur Bekämpfung von Mykosen bei Mensch und Tier sowie zur Bekämpfung von Pilzbefall bei Pflanzen und auf Werkstoffen geeignet.

In diesem Zusammenhang ist unter dem Ausdruck "Halothienyl" ein in 2- oder 3-Stellung verknüpfter Thienylrest, der in beliebiger Position mit einem Halogenatom, unvorzugsweise Brom oder Chlor, substituiert sein kann, unter den Ausdrücken "$C_1$-$C_3$-, $C_1$-$C_4$- und $C_1$-$C_8$-Alkyl" ein unverzweigter oder verzweigter Kohlenwasserstoffrest mit 1-3, 1-4 oder 1-8 C-Atomen, unter dem Ausdruck "$C_3$-$C_6$- bzw. $C_5$-$C_6$-Cycloalkyl" einen Cyclopropyl-, -butyl-, -pentyl- oder Cyclohexyl- bzw. Cyclopentyl- oder Cyclohexyl-Rest, unter dem Ausdruck "$C_1$-$C_4$-Alkoxy" eine Alkoxygruppe, deren Kohlenwasserstoffrest die unter dem Ausdruck "$C_1$-$C_4$-Alkyl" angegebene Bedeutung hat und unter dem Ausdruck "Halogen" ein F-, Cl-, Br- oder J-Atom zu verstehen.

Bevorzugt sind diejenigen Verbindungen der Formel I, in denen mindestens einer der Substituenten bzw. Indices die folgende Bedeutung hat:

A CH oder N,
Ar eine durch 1 oder 2 F- oder Cl-Atome substituierte Phenylgruppe,
$R^1$ $CH_3$ oder $C_2H_5$
g 0 oder 2
Y

zu dem heterocyclischen Rest a)

$R^2$ $C_1$-$C_4$-Alkyl, eine unsubstituierte oder 1 oder 2 gleiche oder verschiedene Substituenten tragende Phenylgruppe, wobei die Substituenten F, Cl, $OCH_3$, $OC_2H_5$, $CH_3$ oder $C_2H_5$ bedeuten, oder eine Benzyl- oder eine durch ein F- oder Cl-Atom im Phenylrest substituierte Benzylgruppe,

$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert, oder $CF_3$,

$R^4$ $C_1$-$C_4$-Alkyl, Benzyl oder

$R^3$ und $R^4$ zusammen $-(CH_2)_3-$, $-(CH_2)_4-$ oder -CH=CH-CH=CH-,

q 0 oder 1

zu dem heterocyclischen Rest b)

$R^5$ H oder CN,

$R^6$ H, $CH_3$ oder Phenyl

$R^7$ H oder $CF_3$

$R^8$ Phenyl oder durch F, Cl, $CH_3$ oder $OCH_3$ substituiertes Phenyl oder, falls $R^5$ CN und/oder $R^7$ $CF_3$ bedeutet, zusätzlich H und

$R^7$ und $R^8$ zusammen $-(CH_2)_4-$

zu dem heterocyclischen Rest c)

$R^9$ H

$R^{10}$ CN, $COOCH_3$ oder $COOC_2H_5$,

$R^{11}$ $C_1$-$C_4$-Alkyl, $C_1$-$C_4$-Alkoxy, F, Cl, Br oder $CF_3$

n 0, 1 oder 2, wobei falls $R^{11}$ $CF_3$ bedeutet n=1 ist und, falls n ungleich 0 ist, $R^{11}$ in 5, 6, 7 oder 8-Stellung stehen kann,

zu dem heterocyclischen Rest d)

$R^{13}$ H, $C_1$-$C_4$-Alkyl oder eine unsubstituierte oder durch 1 oder 2 F, Cl, $OCH_3$ oder $CH_3$ substituierte Phenylgruppe.

Besonders bevorzugt sind Verbindungen der Formel I, in denen mindestens einer der Substituenten bzw. Indizes die folgende Bedeutung hat:

A CH oder N
Ar 2,4-Dichlorphenyl
$R^1$ $CH_3$
g 0 oder 2
Y
zu den heterocyclischen Resten a)
$R^2$ eine unsubstituierte oder 1 oder 2 gleiche oder verschiedene Substituenten tragende Phenylgruppe, wobei die Substituenten Cl, $OCH_3$, $OC_2H_5$ oder $CH_3$ bedeuten, eine Benzyl- oder eine Chlorbenzylgruppe,
$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert,
$R^4$ $C_1$-$C_4$-Alkyl, Benzyl oder
$R^3$ und $R^4$ zusammen $-(CH_2)_3-$, $-(CH_2)_4-$ oder -CH=CH-CH=CH-,
q 0 oder 1,

zu dem heterocyclischen Rest b)
$R^5$ H oder CN,
$R^6$ H oder $CH_3$,
$R^7$ H oder $CF_3$,
$R^8$ Phenyl oder durch Cl oder $OCH_3$ substituiertes Phenyl oder, falls $R^5$ CN und/oder $R^7$ $CF_3$ bedeutet, zusätzlich H,

zu dem heterocyclischen Rest c)
$R^9$ H,
$R^{10}$ CN, $COOCH_3$ oder $COOC_2H_5$,
$R^{11}$ $CH_3$, $C_2H_5$, $OCH_3$, $OC_2H_5$, F, Cl, Br oder $CF_3$,
n 0, 1 oder 2, wobei, falls $R^{11}$ $CF_3$ bedeutet, n=1 ist und, falls n ungleich 0 ist, $R^{11}$ in 5-, 6-, 7- oder 8-Stellung stehen kann,

zu dem heterocyclischen Rest d)
$R^{13}$ eine unsubstituierte oder durch 1 oder 2 F, Cl, $OCH_3$ oder $CH_3$ substituierte Phenylgruppe.

Die Erfindung betrifft weiterhin die möglichen Stereoisomeren der Formel I, sowohl als Diastereomeren-Racemate wie auch als reine Enantiomere, sowie ihre pharmazeutisch akzeptablen Salze. Insbesondere betrifft das die wegen der 2,4-Disubstitution des 1,3-Dioxolan-Rings möglichen Stereoisomeren; die 2-Azolylmethyl-Gruppe kann zum 4-ständigen Substituenten, der substituierten Phenoxymethyl-Gruppe, cis- oder trans-ständig angeordnet sein. Die cis-Isomeren zählen zu den bevorzugten erfindungsgemäßen Verbindungen.

Als Salze der erfindungsgemäßen Verbindungen der Formel I kommen solche mit physiologisch unbedenklichen anorganischen und organischen Säuren, wie beispielsweise Chlor-, Brom- oder Jodwasserstoff-, Schwefel-, Phosphor-, Salpeter-, Benzolsulfon-, Toluolsulfon-, Sulfamin-, Methylschwefel-, Essig-, Propion-, Oel-, Palmitin-, Stearin, Malon-, Malein-, Bernstein-, Glutar-, Aepfel-, Wein-, Zitronen-, Fumar-, Milch-, Glykol-, Brenztrauben-, Benzoe-, Toluyl-, Glutamin-, Furancarbon-, Salicyl- oder Mandelsäure in Betracht. Bevorzugt werden Salze mit physiologisch verträglichen anorganischen Säuren, stark bis mittelstark sauren Derivaten solcher Säuren oder mit Fumarsäure.

Von den bekannten, gegen Pilze und Bakterien wirksamen, oben erwähnten Azolylmethyl-2-aryl-4-[(4-piperazino-phenoxy)-methyl]-1,3-dioxolanen unterscheiden sich die erfindungsgemäßen Verbindungen wesentlich durch die Art der Substituenten an der Piperazinophenoxymethyl-Einheit in der 4-Stellung des Dioxolanrings sowie durch die wahlweise vorhandene zusätzliche Substitution des Phenylrings der 4-ständigen Phenoxygruppe. Von den teilweise ähnlichen, in der EP-A 7 696 erwähnten Verbindungen unterscheiden sich die erfindungsgemäßen Verbindungen entweder durch die Art und Stellung der Substituenten an den Heterocyclen

oder durch die andersartige Struktur der an dem Piperazinring gebundenen heterocyclischen Reste.

Überraschenderweise zeigen die erfindungsgemäßen 2-Azolyl-2-aryl-4-[(4-piperazino-phenoxy)-methyl]-1,3-dioxolane eine breitere und bessere antimykotische Wirkung als die bekannten 2-Azolylmethyl-2-aryl-1,3-dioxolan-Derivate und als das bekannte 2-S,(R)-(2,4-Dichlorphenyl)-2-(imidazol-1-ylmethyl)-4-R,(S)-[4-(4-acetylpiperazin-1-yl)-phenoxy-methyl]-1,3-dioxolan (Ketoconazol).

Die Erfindung betrifft weiterhin ein Verfahren zur Herstellung der Verbindungen der Formel I und ihrer Salze, das dadurch gekennzeichnet ist, daß man
A) eine Verbindung der Formel II,

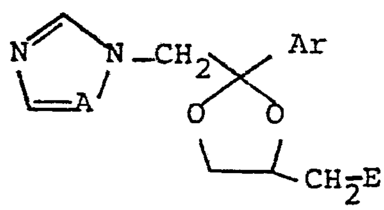

II

in der
A und Ar die zu Formel I angegebenen Bedeutungen haben
und
E Halogen wie F, Cl, Br, J oder Acyloxy, wie Acetoxy, Trifluoracetyloxy, Benzoyloxy, Nitrobenzoyloxy, Alkylsulfonyloxy, wie Methansulfonyloxy, oder Arylsulfonyloxy, wie Benzol-, Nitrobenzol-, Brombenzol- oder Toluolsulfonyloxy, bedeutet,
mit einer Verbindung der Formel III

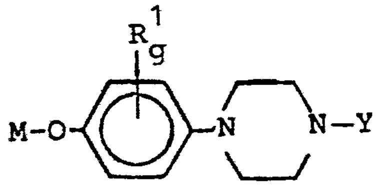

III,

in der

M H, ein Alkali- oder Erdalkalimetall, insbesondere Li, Na oder K, oder $NH_4$ bedeutet und

$R^1$, g und Y die zu Formel I angegebenen Bedeutungen haben, umsetzt, oder daß man

B) eine Verbindung der Formel IV,

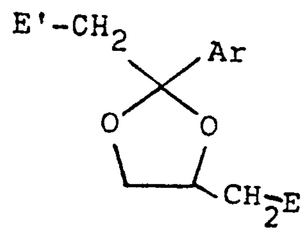

IV

in der

Ar die zu Formel I und E und E' die zu Formel II für E angegebenen Bedeutungen haben,

zunächst mit einer Verbindung der Formel III umsetzt und hierbei eine Verbindung der Formel V herstellt,

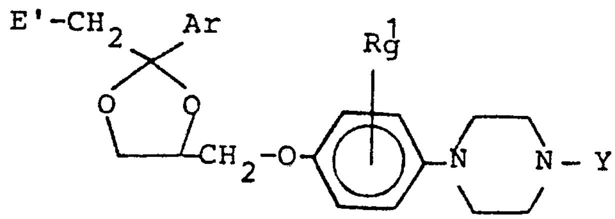

V

in der

Ar, $R^1$, g und Y die zu Formel I und E' die zu Formel II für E angegebenen Bedeutungen haben,

und anschließend eine Verbindung der Formel V mit einer Verbindung der Formel VI umsetzt,

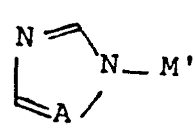

VI

in der A, CH oder N und

M' H, ein Alkali- oder Erdalkalimetall oder $Si(CH_3)_3$ bedeutet,

oder daß man

C) eine Verbindung der Formel VII,

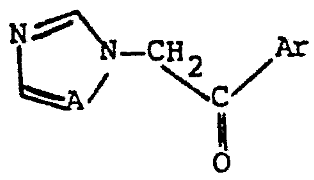

VII

in der A und Ar die zu Formel I angegebenen Bedeutungen haben, mit einem 1,2-Diol der Formel VIII

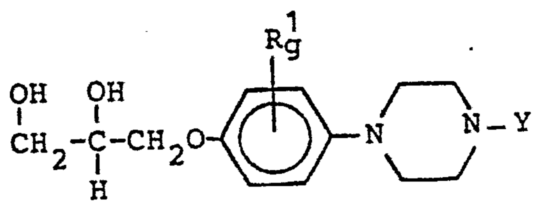

VIII

in der $R^1$, g und Y die zu Formel I angegebenen Bedeutungen haben, umsetzt,

oder daß man

D) eine Verbindung der Formel IX,

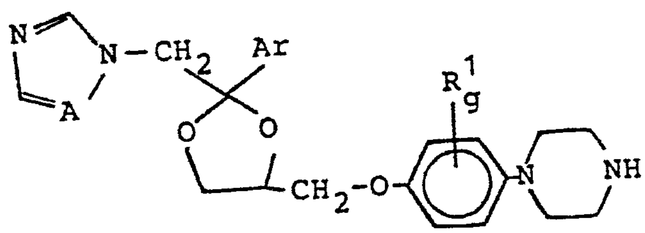

IX

in der A, Ar, $R^1$ und g die zu Formel I angegebenen Bedeutungen haben, mit einer Verbindung der Formel X,

$$E''-Y \qquad X$$

in der E" $C_1$-$C_4$-Alkoxy, Cl, Br, J, Acyloxy, wie Acetyloxy oder Benzoyloxy, Alkylsulfonyloxy, wie Methansulfonyloxy, oder Arylsulfonyloxy, wie Benzol-, Nitrobenzol- oder Toluolsulfonyloxy, bedeutet und Y die zu Formel I unter a, b, c und d angegebenen Bedeutungen hat, umsetzt, und gegebenenfalls die nach Weg A)-D) erhaltenen Verbindungen der Formel I mit anorganischen oder organischen Säuren in ihre physiologisch verträglichen Säureadditionssalze überführt.

In diesem Zusammenhang ist unter dem Ausdruck "Acyloxy" ein geradkettiger oder verzweigter $C_1$-$C_4$-Alkanoyloxy-Rest oder ein im Phenylkern unsubstituierter oder mit bis zu 2 gleichen oder verschiedenen Substituenten substituierter Benzoyloxy-Rest zu verstehen, wobei die Substituenten $CH_3$, $OCH_3$, F, Cl, oder Br bedeuten können, und unter dem Ausdruck "Arylsulfonyloxy" ein unsubstituierter oder ein mit einem Cl, Br, $CH_3$, $C_2H_5$, $OCH_3$, $OC_2H_5$ oder $NO_2$ substituierter Phenylsulfonyloxy- oder ein Naphthylsulfonyloxy-Rest zu verstehen.

Zur Umsetzung mit einer Verbindung der Formel IX werden bevorzugt verwendet entweder

a) eine Verbindung der Formel $Xa_1$ oder der Formel $Xa_2$

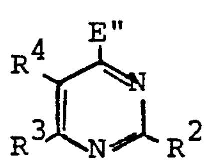

$Xa_1$ ,

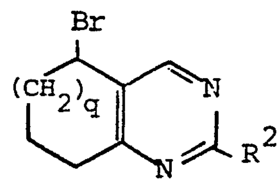

$Xa_2$ ,

in denen $R^2$-$R^4$ und q die zu Formel I angegebenen Bedeutungen haben und E", Cl, Br oder $OCH_3$ bedeutet, oder

b) eine Verbindung der Formel Xb,

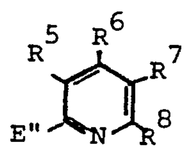

Xb,

in der $R^5$-$R^8$ die zu Formel I angegebenen Bedeutungen haben und E" Cl, $CH_3\underset{\overset{\|}{O}}{C}-O-$, $C_6H_5-\underset{\overset{\|}{O}}{C}-O-$, $CH_3SO_2-O-$, $4-CH_3-C_6H_4-SO_2-O-$ oder $C_6H_5-SO_2O-$ bedeutet, oder

c) eine Verbindung der Formel Xc,

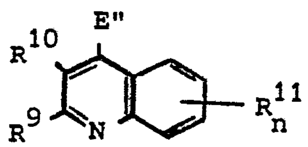

Xc,

in der $R^9$-$R^{11}$ und n die zu Formel I angegebenen Bedeutungen haben und

E" Cl oder Br bedeutet oder

d) eine Verbindung der Formel Xd,

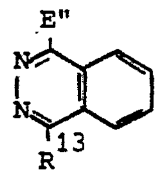

Xd,

in der $R^{13}$ die zu Formel I angegebenen Bedeutungen hat und

E" Cl oder $OCH_3$ bedeutet.

Besonders bevorzugt für die Umsetzung mit einer Verbindung der Formel IX sind diejenigen Verbindungen der Formeln $Xa_1$, Xb, Xc, und Xd, bei denen E" Cl bedeutet.

Die Verfahrens-Variante A), wobei bei den Verbindungen der Formel II E bevorzugt Cl, Br, Acetoxy, Trifluoracetoxy, Methansulfonyloxy oder (subst.) Phenylsulfonyloxy bedeutet, wird bei einer Temperatur zwischen 20°C und 180°C, vorteilhaft zwischen 40°C und 120°C, in Anwesenheit einer Base und zweckmäßig in einem inerten organischen Lösungsmittel, wie beispielsweise N,N-Dimethylformamid, N,N-Dimethylacetamid, Dimethylsulfoxid, N-Methylpyrrolidon-2, 1,2-Dimethoxyethan, Dioxan, Tetrahydrofuran, 4-Methyl-2-pentanon, Methanol, Ethanol, Isopropylalkohol, Propanol, Butanol, Pentanol, Tert.-Butylalkohol, Methylglykol, Methylenchlorid, Acetonitril oder einem aromatischen Kohlenwasserstoff wie Benzol, Chlorbenzol, Toluol oder Xylol durchgeführt. Es können auch Gemische der beispielhaft genannten Lösungsmittel verwendet werden.

Geeignete Basen sind beispielsweise Alkalimetall- oder Erdalkalimetall-carbonate, -hydrogencarbonate, -hydroxide, -amide, -alkoholate oder -hydride wie z.B. Natriumcarbonat, Natriumhydrogencarbonat, Kaliumcarbonat, Natriumhydroxid, -amid oder -methylat, Kalium-t-butylat oder Natriumhydrid, oder organische Basen, z.B. tertiäre Amine wie Triethylamin, Tributylamin, Ethylmorpholin oder Pyridin, Dimethylaminopyridin, Chinolin oder 1,5-Diazabicyclo-[5,4,0]undec-5-en (DBU).

Die Umsetzung kann ebenso unter den Bedingungen einer Phasentransferreaktion ausgeführt werden, indem man die Reaktionspartner in einem geeigneten Lösungsmittel, wie z.B. Ether, Dioxan, Tetrahydrofuran, Methylenchlorid, N,N-Dimethylform- oder -acetamid, N-Methylpyrrolidon-(2), Butanol, tert.-Butanol, einem aliphatischen, cycloaliphatischen oder aromatischen Kohlenwasserstoff wie Hexan, Cyclohexan, Benzol, Toluol oder Xylol, Methylglykol, Anisol oder Chlorbenzol unter kräftigem Rühren in Gegenwart eines Phasentransferkatalysators und entweder einem gepulverten Alkalihydroxid, wie z. B. Natrium- oder Kaliumhydroxid oder einer konzentrierten wäßrigen Lösung derselben, vorzugsweise in einem Temperaturbereich von 20°C bis 120°C aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise 1 bis 12 C-Atomen im Alkylrest oder Kronenether, wie z.B. 12-Crown-4, 15-Crown-5, 18-Crown-6 oder Dibenzo-18-crown-6.

Herstellung der Ausgangsstoffe:

Die Ausgangsverbindungen der Formel II, in der Ar und A die zu Formel I angegebenen Bedeutungen haben, sind teilweise bekannt; die nicht bekannten können in Analogie zu den bekannten hergestellt werden.

Die Verfahrens-Variante B), wobei eine Verbindung der Formel IV, in der E bevorzugt Br, J oder Trifluoracetyloxy, Methansulfonyloxy, Benzol-, Nitrobenzol-, Brombenzol- oder Toluolsulfonyloxy und E' bevorzugt Cl oder Br bedeuten, mit einer Verbindung der Formel III, in der M, $R^1$, g und Y die angegebenen Bedeutungen haben, unter Bildung einer Verbindung der Formel V umgesetzt wird, wird unter den gleichen Reaktionsbedingungen wie bei Variante A zur Herstellung von Verbindungen der Formel I angegeben durchgeführt.

Die Herstellung von Verbindungen der Formel I durch Umsetzung von Verbindungen der Formel V mit Verbindungen der Formel VI erfolgt zweckmäßig in einem inerten Lösungsmittel in Gegenwart einer Base, wie zum ersten Herstellverfahren vorstehend angegeben, vorzugsweise in einem Temperaturbereich von 100 bis 190°C. Die Umsetzung erfolgt zweckmäßigerweise in Anwesenheit eines Alkalijodides wie z.B. Natrium- oder Kaliumjodid, gegebenenfalls in einem Autoklaven unter Druck.

Die vorstehend beschriebenen Umsetzungen können zweckmäßig als Eintopfreaktion durchgeführt werden, indem man zunächst bei 40 bis 100°C eine Verbindung der Formel VI mit einer Verbindung der Formel III in Gegenwart einer Base in einem inerten Lösungsmittel umsetzt. Anschliessend wird ohne Isolierung der Verbindung der Formel V eine Verbindung der Formel VI und gegebenenfalls ein weiteres Moläquivalent einer Base und ein Alkalijodid (z.B. Kaliumjodid) zugegeben und auf 100 bis 190°C erhitzt.

Herstellung der Ausgangsstoffe:

Verbindungen der Formel IV, in der E und E' die zur Formel II für E angegebenen Bedeutungen haben, sind bekannt. Sie werden hergestellt, indem man die Hydroxymethylgruppe in einer Verbindung der Formel

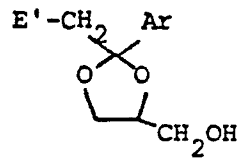

(XI)

XI in üblicher Weise in eine reaktionsfähige Estergruppe überführt. So werden z.B. die Verbindungen der Formel IV, in der E' bevorzugt Cl oder Br und E Methansulfonyloxy bedeuten, hergestellt, indem man eine Verbindung der Formel XI, in der Ar die zu Formel I angegebenen Bedeutungen hat und E' Cl oder Br bedeuten, mit Methansulfochlorid bei -10°C bis +50°C zweckmäßig in einem inerten Lösungsmittel, in Gegenwart einer Base umsetzt. Verbindungen der Formel IV, in der E beispielsweise Brom bedeutet, werden durch Umsetzung von Verbindungen der Formel XI (E' = Cl oder Br) mit Bromierungsmitteln, wie z.B. $PBr_3$ oder $POBr_3$ in einem inerten Lösungsmittel bei 0°C bis 100°C hergestellt. Derartige Verbindungen können auch hergestellt werden, indem man eine Verbindung der Formel XII,

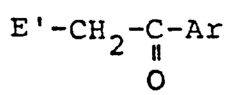

(XII)

in der E' Cl oder Br bedeutet und Ar die angegebenen Bedeutungen hat, mit 1-Brom-2,3-propandiol in einem inerten Lösungsmittel in Gegenwart einer starken Säure unter Bildung eines 1,3-Dioxolans nach für derartige Ketalisierungen bekannten Methoden umsetzt.

Die Verbindungen der Formel XI sind bekannt.

Die Verfahrens-Variante C), wobei man eine Verbindung der Formel VII mit einer Verbindung der Formel VIII unter Bildung einer Verbindung der Formel I umsetzt, wird im allgemeinen unter den gleichen Bedingungen durchgeführt, wie

die Herstellung von Verbindungen der Formel IV (Variante B). Die Ketalisierung von Ketonen der Formel VII mit Glycerin-Derivaten der Formel VIII erfolgt vorteilhaft in einem Lösungsmittelgemisch, bestehend aus einem inerten Lösungsmittel, das mit Wasser ein azeotropes Gemisch bildet, wie z.B. Benzol, Toluol, Xylol, Chlorbenzol oder Cyclohexan, und einem Alkohol, in Anwesenheit einer starken Säure in einem Temperaturbereich von 75 bis 180°C. Vorteilhaft werden bei dieser Ketalisierung mindestens 2,5 Äquivalente einer starken Säure (bez. auf die Azolverbindung der Formel VII) und als Alkohole aliphatische Alkohole mit einem Kp zwischen 75° und 150°C und/oder Monoether von niederen Diolen, die zwischen 100° und 150°C sieden, verwendet.

Herstellung der Ausgangsstoffe:

Die Verbindungen der Formel VII sind bekannt und können nach bekannten Methoden hergestellt werden.

Verbindungen der Formel VIII, in der $R^1$, g und Y die zu Formel I angegebenen Bedeutungen haben, werden durch Umsetzung von Verbindungen der Formel III mit 1-Halogen-2,3-propandiol hergestellt, in analoger Weise wie in Org. Synth. Collect. Vol. I, S. 296 beschrieben.

Bei der Verfahrens-Variante D) wird eine Verbindung der Formel IX mit einer heterocyclischen Verbindung der Formel X, bevorzugt mit einer heterocyclischen Verbindung entweder der Formel $Xa_1$ oder $Xa_2$ oder der Formel Xb, Xc oder Xd, in denen E" jeweils die angegebenen Bedeutungen hat, zweckmäßig in einem inerten organischen Lösungsmittel in einem Temperaturbereich von 20° bis 180°C, vorzugsweise von 50° bis 120°C, umgesetzt. Diese Umsetzung wird vorteilhaft in Gegenwart einer Base, die vorzugsweise in äquimolarer Menge angewandt wird, durchgeführt.

Die Synthese von Verbindungen der Formel I aus den Verbindungen der Formeln IX und X kann auch ohne Basenzusatz ausgeführt werden. Die Reaktanten der Formeln IX und X können in unterschiedlichen Molverhältnissen angewendet werden, d.h. es können entweder jeweils die Verbindungen der Formel IX oder diejenigen der Formel X im Überschuß angewandt werden, vorteilthaft werden äquimolare Mengen angewendet.

Als Lösungsmittel kommen beispielsweise Kohlenwasserstoffe, allgemein Ether wie Diethylether, 1,2-Dimethoxyethan, Tetrahydrofuran oder Dioxan, oder Acetonitril, Butyronitril, Dimethylform- oder -acetamid, Aceton, 4-Methyl-2-pentanon, Methylenchlorid, Chloroform, Dimethylsulfoxid, Anisol, Chlorbenzol, Tetrachlorethen oder Gemische dieser Lösungsmittel in Frage.

Geeignete Basen sind die beispielhaft bei der Verfahrens-Variante A) erläuterten.

Die Umsetzung kann ebenso unter den Bedingungen einer Phasentransferreaktion, wie sie bei der Beschreibung der Verfahrens-Variante A) erläutert sind, ausgeführt werden.

Herstellung der Ausgangsstoffe:

Die Verbindungen der Formel IX sind z.T. bekannt (vgl. DOS 2 804 096, z.B. Beispiel 21); solche, die von den bekannten abweichende Bedeutungen für Ar haben und/oder bei denen g 1 oder 2 bedeutet, können in Analogie zu den bekannten (vgl. DOS 2 804 096) hergestellt werden.

Die Verbindungen der Formel X, in der E" und Y die angegebenen Bedeutungen haben, sind teilweise bekannt. Das trifft vor allem für Verbindungen der Formel X mit E" = Cl zu.

Sofern die Substituenten an den heterocyclischen Verbindungen $Xa_1$, Xb, Xc und Xd von bekannten Verbindungen abweichen und es sich bei den Substituenten um weitgehend inerte, also unempfindliche handelt, können diese Verbindungen der Formeln $Xa_1$-Xd, in denen die Substituenten $R^2$-$R^{13}$ und n die angegebenen Bedeutungen haben, in Analogie zu den bekannten Verbindungen dieser Strukturen hergestellt werden.

Die Verbindungen $Xa_2$ mit q=0 oder 1 und $R^2$ wie zu Formel I angegeben, werden aus Verbindungen der Formel XI,

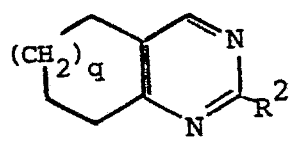

XI,

in der q und $R^2$ die angegebenen Bedeutungen haben, durch Bromierung mit N-Brom-succinimid hergestellt.

Verbindungen der Formel Xc, in der $R^{10}$ $COOR^{12}$ und E" z.B. Cl bedeuten, werden ebenfalls gemäß den zur Herstellung von Verbindungen der Formel Xc üblichen Methoden, nämlich durch Umsetzung von Verbindungen der Formel XII,

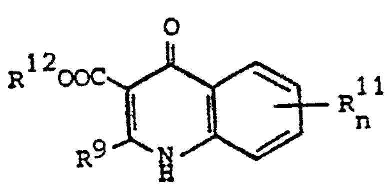

XII,

in der $R^9$, $R^{11}$, $R^{12}$ und n die angegebenen Bedeutungen haben, mit $POCl_3$ oder $SOCl_2$ hergestellt.

Verbindungen der Formeln $Xa_1$ und Xd, in denen E" $OCH_3$ bedeutet, werden aus entsprechenden Verbindungen der Formeln $Xa_1$ und Xd, in denen E" z. B. Cl bedeutet, durch Umsetzung mit Methanol oder mit Methanol plus bis zu einem Äquivalent Alkalimethanolat hergestellt. Als Alkalimetalle werden Li, Na oder K verwendet.

Die Reaktionszeiten betragen je nach Verfahrensvariante und je nach Temperaturbereich wenige Minuten bis einige Stunden.

Falls erforderlich, kann eine Reinigung der Verfahrenserzeugnisse durch Umkristallisation aus einem geeigneten Lösungsmittel oder Lösungsmittelgemisch oder durch Säulenchromatographie an Kieselgel erfolgen.

Die diastereomeren Racemate (cis- oder trans-Form) der Verbindungen der Formel I können in üblicher Weise, z.B. durch selektive, fraktionierte Kristallisation oder Säulenchromatographie getrennt werden.

Da die stereochemische Konfiguration bereits in den Zwischenprodukten der Formel II vorgegeben ist, kann die Trennung in die cis- und trans-Form bereits auf dieser Stufe oder noch früher, beispielsweise auf der Stufe der Zwischenprodukte der allgemeinen Formel IV, oder bei den Zwischenprodukten der Formel IX erfolgen.

Die cis- und trans-diastereomeren Racemate können ihrerseits in üblicher Weise in ihre optischen Antipoden cis(+), cis(-) bzw. trans(+) und trans(-) getrennt werden.

Bevorzugt werden zur Herstellung von Verbindungen I die Verfahrens-Varianten A, B und D angewendet.

Die Erfindung betrifft weiterhin Verbindungen der Formel IIIa,

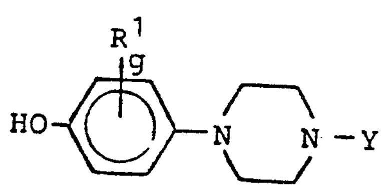

IIIa,

in der bedeuten:

$R^1$ $C_1$-$C_3$-Alkyl, F oder Cl,

g 0, 1 oder 2 und

Y die folgenden heterocyclischen Reste

a) 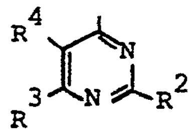 oder 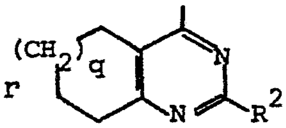 , in denen

$R^2$ $C_1$-$C_4$-Alkyl, eine unsubstituierte oder 1 oder 2 Substituenten tragende Phenylgruppe, wobei die Substituenten gleich oder verschieden sein können und Halogen, Trifluormethyl, Methoxy, Ethoxy, Nitro oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Methoxy-, Ethoxy- oder $C_1$-$C_3$-Alkyl bedeuten,

$R^3$ H, $C_1$-$C_8$-Alkyl, $C_3$-$C_6$-Cycloalkyl-$C_1$-$C_3$-alkyl, eine unsubstituierte oder 1 oder 2 Substituenten tragende Phenylgruppe, wobei die Substituenten gleich oder verschieden sein können und Halogen, Methoxy-, Ethoxy oder $C_1$-$C_3$-Alkyl bedeuten, eine unsubstituierte oder im Phenylrest durch Methoxy, 1,2-Methylendioxy, F, Cl oder $C_1$-$C_3$-Alkyl substituierte Phenyl-$C_1$-$C_2$-alkylgruppe, oder Trifluormethyl,

$R^4$ H, $C_1$-$C_4$-Alkyl oder Benzyl, oder $R^3$ und $R^4$ zusammen $-(CH_2)_r-$, wobei r=3 oder 4 ist, oder -CH=CH-CH=CH-,

q 0 oder 1 bedeutet, oder

b) 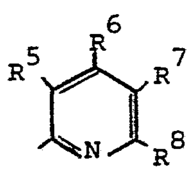 , in dem

$R^5$ H oder CN

$R^6$ H, $C_1$-$C_4$-Alkyl oder eine unsubstituierte oder durch $OCH_3$, F, Cl, $CH_3$ oder $C_2H_5$ substituierte Phenylgruppe,

$R^7$ H, Benzyl oder $CF_3$,

$R^8$ $C_5$-$C_6$-Cycloalkyl oder eine unsubstituierte oder

durch $OCH_3$, F, Cl, $CH_3$ oder $C_2H_5$ substituierte Phenylgruppe bedeuten und, falls $R^5$ CN und/oder $R^7$ $CF_3$ bedeutet, $R^8$ auch H bedeuten kann, oder

$R^7$ und $R^8$ zusammen $-(CH_2)_4-$ bedeuten, oder

c) 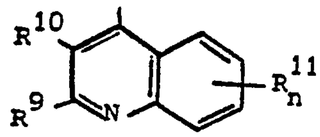 , in dem

$R^9$ H, Methyl oder Ethyl,

$R^{10}$ H, CN oder $COOR^{12}$, wobei $R^{12}$ Methyl oder Ethyl bedeutet,

$R^{11}$ $C_1$-$C_4$-Alkyl, $C_1$-$C_4$-Alkoxy, Halogen oder Trifluormethyl und

n 0, 1 oder 2 bedeuten, wobei, falls $R^{11}$ $CF_3$ bedeutet, n=1 ist, und, falls n ungleich 0, die Reste $R^{11}$ in 5-, 6-, 7- oder 8-Stellung des Chinolinsystems stehen können, oder

d) 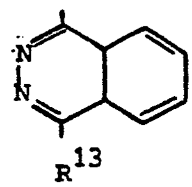 , in dem

$R^{13}$ H, $C_1$-$C_4$-Alkyl oder eine unsubstituierte oder 1 oder 2 Substituenten tragende Phenylgruppe, wobei die Substituenten gleich oder verschieden sein können und Halogen, Methoxy, Ethoxy, Methyl oder Ethyl bedeuten,

sowie deren Säureadditionssalze.

Bevorzugt werden Verbindungen der Formel IIIa, bei denen mindestens einer der Substituenten oder Indices $R^1$, g, Y, $R^2$-$R^{13}$, q und n die folgenden Bedeutungen hat:

$R^1$ $CH_3$ oder $C_2H_5$

g 0 oder 2

Y

zu dem heterocyclischen Rest a)

$R^2$ $C_1$-$C_4$-Alkyl, eine unsubstituierte oder 1 oder 2 gleiche oder verschiedene Substituenten tragende Phenylgruppe, wobei die Substituenten F, Cl, $OCH_3$, $OC_2H_5$, $CH_3$ oder $C_2H_5$ bedeuten, oder eine Benzyl- oder eine durch ein F- oder Cl-Atom im Phenylrest substituierte Benzylgruppe,

$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert, oder $CF_3$,

$R^4$ $C_1$-$C_4$-Alkyl, Benzyl oder

$R^3$ und $R^4$ zusammen $-(CH_2)_3-$, $-(CH_2)_4-$ oder -CH=CH-CH=CH-,

q O oder 1

zu dem heterocyclischen Rest b)

$R^5$ H oder CN,

$R^6$ H, $CH_3$ oder Phenyl

$R^7$ H, $CH_3$ oder $CF_3$

$R^8$ H, $CH_3$, $C_5$-$C_6$-Cycloalkyl, Phenyl oder durch F, Cl oder $OCH_3$ substituiertes Phenyl und/oder falls $R^5$ CN und/oder $R^7$ $CF_3$ bedeutet, $R^8$ auch H oder

$R^7$ und $R^8$ zusammen $-(CH_2)_4-$

zu dem heterocyclischen Rest c)

$R^9$ H

$R^{10}$ CN, $COOCH_3$ oder $COOC_2H_5$,

$R^{11}$ $C_1$-$C_4$-Alkyl, $C_1$-$C_4$-Alkoxy, F, Cl, Br oder $CF_3$

n O, 1 oder 2, wobei falls $R^{11}$ $CF_3$ bedeutet, n=1 ist und, falls n ungleich O ist, $R^{11}$ in 5-, 6-, 7- oder 8-Stellung stehen kann,

zu dem heterocyclischen Rest d)

$R^{13}$ H, $C_1$-$C_4$-Alkyl oder eine unsubstituierte oder durch 1 oder 2 F, Cl, $OCH_3$ oder $CH_3$ substituierte Phenylgruppe

Besonders bevorzugte Verbindungen der Formel IIIa sind solche, bei denen mindestens einer der Substituenten oder Indices bedeutet:

0237963

$R^1$ $CH_3$

g 0 oder 2

Y

zu den heterocyclischen Resten a)

$R^2$ eine unsubstituierte oder 1 oder 2 gleiche oder verschiedene Substituenten tragende Phenylgruppe, wobei die Substituenten Cl, $OCH_3$, $OC_2H_5$ oder $CH_3$ bedeuten, eine Benzyl- oder eine Chlorbenzylgruppe,

$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert,

$R^4$ $C_1$-$C_4$-Alkyl, Benzyl oder

$R^3$ und $R^4$ zusammen $-(CH_2)_3-$, $-(CH_2)_4-$ oder -CH=CH-CH=CH-,

q 0 oder 1,

zu dem heterocyclischen Rest b)

$R^5$ H oder CN,

$R^6$ H, $CH_3$ oder $C_2H_5$,

$R^7$ H oder $CF_3$,

$R^8$ $C_5$-$C_6$-Cycloalkyl, Phenyl oder durch Cl oder $OCH_3$ substituiertes Phenyl oder, falls $R^5$ CN und/oder $R^7$ $CF_3$ bedeutet, zusätzlich H,

zu dem heterocyclischen Rest c)

$R^9$ H,

$R^{10}$ CN, $COOCH_3$ oder $COOC_2H_5$,

$R^{11}$ $CH_3$, $C_2H_5$, $OCH_3$, $OC_2H_5$, F, Cl, Br oder $CF_3$,

n 0, 1 oder 2, wobei, falls $R^{11}$ $CF_3$ bedeutet, n=1 ist und, falls n ungleich 0 ist, $R^{11}$ in 5-, 6-, 7- oder 8-Stellung stehen kann,

zu dem heterocyclischen Rest d)

$R^{13}$ eine unsubstituierte oder durch 1 oder 2 F, Cl, $OCH_3$ oder $CH_3$ substituierte Phenylgruppe.

Die Verbindungen der Formel IIIa, in denen $R^1$, g und Y die angegebenen Bedeutungen haben, sind neu und stellen

wertvolle Zwischenprodukte zur Herstellung der stark antimykotisch sowie fungizid wirkenden Verbindungen der Formel I dar. Ein Teil der Verbindungen der Formel IIIa wirkt ebenfalls antimykotisch sowie fungizid. Ein Teil der Verbindungen der Formel IIIa zeigt darüberhinaus pharmakologische Wirkungen, so z.B. Wirkungen auf das Herz-Kreislaufsystem u.a. durch günstige Beeinflussung des Blutdrucks.

Die Erfindung betrifft außerdem ein Verfahren zur Herstellung von Verbindungen der Formel IIIa, das dadurch gekennzeichnet ist, daß man eine Verbindung der Formel XIII,

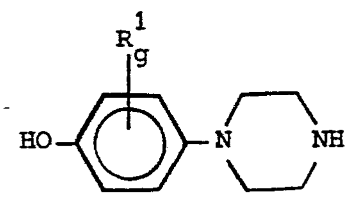

XIII,

in der $R^1$ und g die zu Formel I angegebenen Bedeutungen haben, oder ein Salz dieser Verbindung, mit einer Verbindung der Formel X,

E"-Y     X,

in der E" $C_1$-$C_4$-Alkoxy, Cl, Br, J, Acyloxy, wie Acetoxy oder Benzoyloxy, oder Alkylsulfonyloxy, wie Methansulfonyloxy, oder Arylsulfonyloxy, wie Benzol-, Nitrobenzol- oder Toluolsulfonyloxy bedeutet, und

Y die zur Formel I angegebenen Bedeutungen hat,

umsetzt und gegebenenfalls die erhaltenen Verbindungen der Formel IIIa mit anorganischen oder organischen Säuren in ihre Säureadditionssalze überführt.

Bevorzugt verwendet werden für die erfindungsgemäße Umsetzung mit Verbindungen der Formel XIII entweder

a) eine Verbindung der Formel $Xa_1$ oder der Formel $Xa_2$,

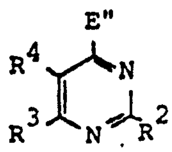

Xa1,

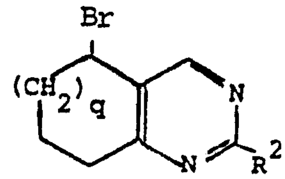

Xa2,

in denen $R^2$-$R^4$ und q die zu Formel I angegebenen Bedeutungen haben, und

E" Cl, Br oder $OCH_3$ bedeutet, oder

b) eine Verbindung der Formel Xb,

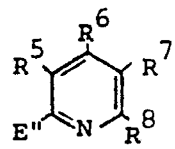

Xb,

in der $R^5$-$R^8$ die zu Formel I angegebenen Bedeutungen haben und E" Cl, $CH_3\underset{\underset{O}{\parallel}}{C}-O-$, $C_6H_5-\underset{\underset{O}{\parallel}}{C}-O-$, $CH_3-SO_2-O-$, $4-CH_3-C_6H_4-SO_2-O-$ oder $C_6H_5-SO_2-O-$ bedeutet, oder

c) eine Verbindung der Formel Xc,

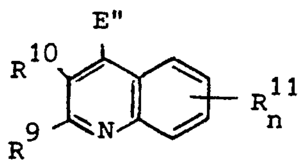

Xc,

in der $R^9$-$R^{11}$ und n die zu Formel I angegebenen Bedeutungen haben und

E" Cl oder Br bedeutet oder

d) eine Verbindung der Formel Xd,

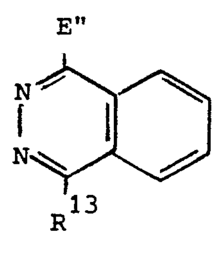

Xd,

in der $R^{13}$ die zu Formel I angegebenen Bedeutungen hat und E" Cl bedeutet.

Besonders bevorzugt für die Umsetzung mit einer Verbindung der Formel XIII sind diejenigen Verbindungen der Formeln $Xa_1$, Xb, Xc und Xd, bei denen E" Cl bedeutet.

Das erfindungsgemäße Verfahren zur Herstellung von Verbindungen der Formel IIIa wird zweckmäßig in einem inerten organischen Lösungsmittel in einem Temperaturbereich von 20° bis 180°C, vorzugsweise von 50° bis 120°C, vorteilhaft in Gegenwart einer Base, die vorzugsweise in äquivalenter Menge angewandt wird, durchgeführt. Werden für das Verfahren Salze der Verbindungen der Formel XIII verwandt, dann wird die dem Salzanteil entsprechende stöchiometrische Menge an Base zugesetzt. Wahlweise kann darüber hinaus dann ein weiterer Anteil an Base angewandt werden.
Die Synthese von Verbindungen der Formel IIIa aus den Verbindungen der Formeln XIII und X kann, sofern die Verbindungen XIII nicht als Salz angewandt werden, auch ohne Basenzusatz ausgeführt werden. Die Reaktanten der Formeln XIII und X können in unterschiedlichen Molverhältnissen angewandt werden, d.h. es können entweder jeweils die Verbindungen der Formel XIII oder diejenigen der Formel X im Überschuß angewandt werden, vorteilhaft werden äquimolare Mengen angewandt.

Als Lösungsmittel kommen beispielsweise Kohlenwasserstoffe, allgemein Ether, wie Diethylether, 1,2-Dimethoxyethan, Tetrahydrofuran oder Dioxan, oder Acetonitril, Butyronitril, Dimethylform- oder -acetamid, Aceton, 4-Methyl-2-pentanon, Methylenchlorid, Chloroform, Dimethylsulfoxid, Anisol, Chlorbenzol, Tetrachlorethen oder Gemische dieser Lösungsmittel in Frage.

Geeignete Basen sind die beispielhaft bei der Verfahrens-Variante A) erläuterten.

Die Reaktionszeiten betragen je nach Temperaturbereich wenige Minuten bis einige Stunden.

Falls erforderlich, kann eine Reinigung der Verfahrenserzeugnisse durch Umkristallisation aus einem geeigneten Lösungsmittel oder Lösungsmittelgemisch oder durch Säulenchromatographie an Kieselgel erfolgen.

Herstellung der Ausgangsstoffe:

Die Verbindung der Formel XIII mit g=0 ist bekannt. Verbindungen der Formel XIII, bei denen g 1 oder 2 bedeutet und $R^1$ die zu Formel I angegebenen Bedeutungen hat, werden in Analogie zu den bekannten Verbindungen durch Umsetzung von entsprechenden 4-Methoxy-Anilinen mit Bis-(2-Chlorethyl)-amin und anschließender Phenolether-Spaltung mit konz. wäßriger Bromwasserstoffsäure hergestellt. Die Herstellung der Verbindungen der Formel X, in der E" und Y die angegebenen Bedeutungen haben, sofern sie nicht bekannt sind, ist bereits bei Verfahrensvariante D) beschrieben.

Die Verbindungen der Formel I und ihre Säureadditionssalze sind wertvolle Arzneimittel. Sie wirken antimikrobiell und eignen sich insbesondere zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt,

z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

0237963

Die therapeutisch wirksamen Verbindungen sollen in den oben aufgeführten pharmazeutischen Zubereitungen zweckmäßig in einer Konzentration von etwa 0,01 bis 99,0, vorzugsweise von etwa 0,05 bis 50 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,05 bis etwa 200, vorzugsweise 0,1 bis 100, insbesondere 0,5 bis 30 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen der Formel I eignen sich auch zur Behandlung von Protozoenerkrankungen beim Menschen und Tier wie sie z. B. durch Infektion mit Trichomonas vaginalis und Entamoeba histolytica sowie durch Trypanosoma cruzi und leishmania donovani hervorgerufen werden.

Die neuen Verbindungen können oral oder lokal angewendet werden. Die orale Anwendung erfolgt in pharmazeutisch üblichen Zubereitungen, z. B. in Form von Tabletten oder Kapseln.

Die Verbindungen der Formel I sind auch als Biozide wirksam. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und echte Mehltaupilze im Obst-, Gemüse-, Getreide- und Zierpflanzenbau.

Die neuen Verbindungen der Formel I eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Die nachfolgenden Beispiele dienen zur näheren Erläuterung der Erfindung, ohne dieselbe einzuschränken.

## Beispiele für das Herstellungsverfahren Variante A):

### Beispiel 1

Eine Mischung aus 1,51 g (3,7 mMol) 1-(4-Hydroxy-3,5-dimethylphenyl)-4-(6-(2-cyclopentyl-ethyl)-2-ethyl-pyrimidin-4-yl)-piperazin, 1,51 g (3,7 mMol) 2-S,(R)-(2,4-Dichlorphenyl)-2-(imidazol-1-ylmethyl)-4-R,(S)-methansulfonyloxymethyl-1,3-dioxolan (cis-Form), 0,28 g Tetrabutylammoniumbromid, 33 ml Toluol und 5,5 ml 50 %ige Natronlauge wurde 3,5 Stunden bei 100°C intensiv gerührt. Danach trennte man bei Raumtemperatur die Phasen, schüttelte die konzentrierte NaOH zweimal mit Ether aus, vereinigte Toluol- und Etherphasen und schüttelte diese dreimal mit Wasser aus. Die Toluol-Ether-Lösung wurde getrocknet, filtriert und im Vakuum am Rotationsverdampfer eingedampft. Der Rückstand (3,20 g) wurde an einer KieselgelS/$CH_2Cl_2$-Säule (ø = 2,0 cm, Höhe 33 cm) unter Elution mit $CH_2Cl_2$ und $CH_2Cl_2/C_2H_5OH$-Mischungen mit steigendem $C_2H_5OH$-Gehalt (bis max. 4 Vol.%) chromatographiert. Nach Elution von Vorzonen (Gehalt 0,25 g) wurden DC-einheitliche Fraktionen vereinigt und im Vakuum eingedampft. Man erhielt 2,15 g DC-reines (= 81 % Ausbeute) 2-S,(R)-(2,4-Dichlorphenyl)-2-(imidazol-1-ylmethyl)-4-R,(S)-[(4-(4-(6-(2-cyclopentyl-ethyl)-2-ethyl-pyrimidin-4-yl)-piperazin-1-yl)-2,6-dimethylphenoxy)-methyl]-1,3-dioxolan (cis-Form) als hochviskoses Öl; Analyse: $C_{39}H_{48}Cl_2N_6O_3$ (MG 719,78)
Ber. C 65.08, H 6.72, N 11.68; Gef. C 65,0; H 7.1, N 11.5 %.

Beispiel 2

Zu einer Lösung von 1,803 g (5 mMol) 1-(4-Hydroxyphenyl)-4-(5,6-dimethyl-2-phenyl-pyrimidin-4-yl)-piperazin in 20 ml abs. N,N-Dimethylformamid (DMF) wurden bei Raumtemperatur 154 mg (5.13 mMol) 80 %ige Natriumhydrid-Öl-Dispersion zugesetzt. Nach Abklingen der Wasserstoffentwicklung wurde eine Lösung von 2.10 g (5.14 mMol) 2-S,(R)-(2,4-Dichlorphenyl)-2-(1,2,4-triazol-1-ylmethyl)-4-R,(S)-methansulfonyloxymethyl-1,3-dioxolan (cis-Form) in 15 ml abs. DMF zugegeben und die Mischung 3 Stunden bei 95 - 97°C gerührt. Anschließend destillierte man das DMF unter Vakuum (3-10 mbar) an einem Rotationsverdampfer ab, versetzte den Rückstand mit 50 ml Wasser und 50 ml $CH_2Cl_2$, schüttelte durch, trennte die Phasen und extrahierte die wäßrige Phase dreimal mit $CH_2Cl_2$. Die vereinigten $CH_2Cl_2$-Extrakte wurden mit $MgSO_4$ getrocknet, filtriert und im Vakuum eingedampft. Der verbliebene Rückstand (3,9 g) wurde an einer Kieselgel S/$CH_2Cl_2$-Säule (⌀ 2,0, Höhe 30 cm) unter Elution mit $CH_2Cl_2$ und $CH_2Cl_2$/$C_2H_5OH$-Mischungen, mit steigendem $C_2H_5OH$-Gehalt (bis max. 2 Vol.%) chromatographiert. Nach Elution von Vorzonen (Gehalt 0,95 g) wurden DC-einheitliche Fraktionen vereinigt und im Vakuum eingedampft. Man erhielt 2,31 g (= 69 % Ausbeute ) 2-S,(R)-(2,4-Dichlorphenyl)-2-(1,2,4-triazol-1-ylmethyl)-4-R,(S)-[4-(4-(5,6-dimethyl-2-phenyl-pyrimidin-4-yl)-piperazin-1-yl)-phenoxymethyl]-1,3-dioxolan (cis-Form) als hochviskoses Öl;

Analyse: $C_{35}H_{35}Cl_2N_7O_3$ (MG 672.64)

Ber. C 62.50, H 5.25, Cl 10.54, N 14.58;

Gef. C 61.8 , H 5.3 , Cl 11.0 , N 14.3 %.

Beispiel 3

Nach der gleichen Arbeitsweise wie im Beispiel 1 beschrieben wurden ausgehend von IIb bzw. IIc (vgl. Tabelle 1) und jeweils der entsprechenden Verbindung IIIa (Y vgl. Tabelle 1) die in der Tabelle 1 aufgeführten Verbindungen der Formel I (g= 0 oder 2, $R^1$ H oder $CH_3$ in 2,6-Stellung) hergestellt.

Tabelle 1

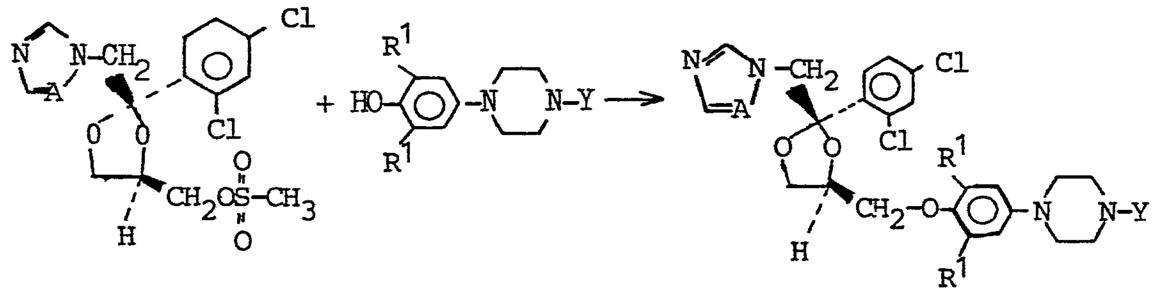

IIb (A = CH) IIIa I

IIc (A = N) (Ar= 2,4-Dichlorphenyl)

| Verb. Nr. | A | $R^1$ | Y | Analyse % Ber. | Gef. | Fp. [°C] | 2,4-Isomere *) |
|---|---|---|---|---|---|---|---|
| 1.1 | CH | H | 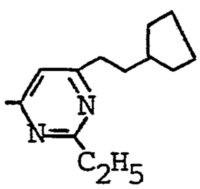 | C 64.25<br>H 6.41<br>N 12.15 | 64.7<br>6.6<br>12.0 | - | cis |
| 1.2 | N | $CH_3$ | 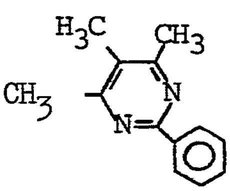 | C 63.42<br>H 5.61<br>Cl 10.12<br>N 13.99 | 62.8<br>5.6<br>10.8<br>13.8 | - | cis |
| 1.3 | N | H | 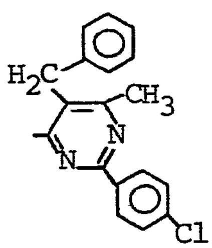 | C 62.88<br>H 4.89<br>N 12.52 | 62.9<br>4.8<br>12.6 | 167-68 | cis |
| 1.4 | CH | H | 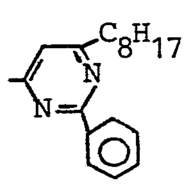 | C 66.74<br>H 6.40<br>N 11.12 | 65.7<br>6.3<br>10.7 | - | cis |

Tabelle 1 (Fortsetzung)

| Verb. Nr. | A | $R^1$ | Y | Analyse % Ber. | Gef. | Fp. [°C] | 2,4-Isomere *) |
|---|---|---|---|---|---|---|---|
| 1.5 | CH | H | 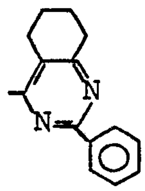 | C 65.42<br>H 5.49<br>N 12.05 | 64.9<br>5.4<br>11.8 | 119-20 | cis |
| 1.6 | CH | H | 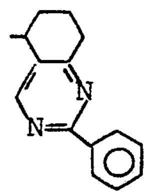 | C 65.42<br>H 5.49<br>N 12.05 | 65.8<br>5.7<br>12.1 | 96-97 | cis |
| 1.7 | CH | H | 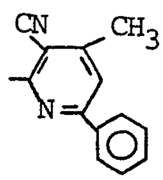 | C 65.20<br>H 5.03<br>N 12.22 | 65.4<br>5.2<br>12.0 | 127-28 | cis |
| 1.8 | CH | H | 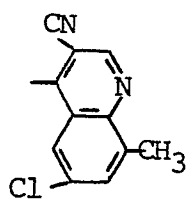 | C 60.92<br>H 4.53<br>N 12.18 | 60.4<br>4.7<br>11.6 | 208-09 | cis |

*) Cis- und Trans- bezieht sich auf den Azolylmethyl-Rest und den (subst.)-Oxymethyl-Rest in der 2- bzw. der 4-Stellung des Dioxolanringes

Beispiel 4

Nach der gleichen Arbeitsweise wie im Beispiel 2 beschrieben wurden ausgehend von IIb bzw. IIc (vgl. Tabelle 1) und jeweils der entsprechenden Verbindung IIIa (Y vgl. Tab. 2) die in der Tabelle 2 aufgeführten Verbindungen der Formel I (g=0 oder 2, $R^1$= H oder $CH_3$ in 2,6-Stellung; cis-Form) hergestellt.

Sofern nach dem Abdestillieren des DMF und Aufnehmen des Rückstands in Wasser ein kristallines Produkt anfiel oder

der $CH_2Cl_2$-Extraktrückstand kristallisierte, wurden diese Verbindungen durch Umkristallisation aus Methanol oder Acetonitril gereinigt. In dieser Weise isolierte Verbindungen sind mit (*) gekennzeichnet. In allen anderen Fällen wurden die in der Tabelle 2 aufgeführten Verbindungen durch Säulenchromatographie wie im Beispiel 2 beschrieben gewonnen.

<u>Tabelle 2</u>

IIb bzw. IIc + 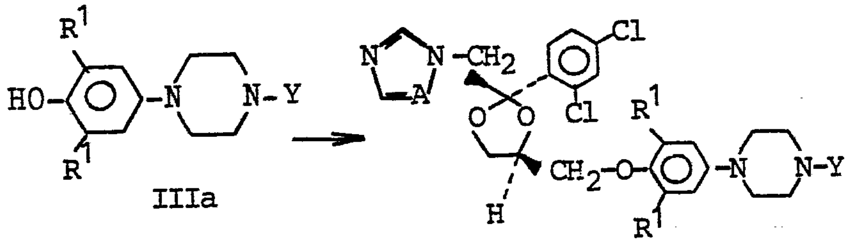

IIIa

I (Ar= 2,4-Dichlorphenyl)

| Verb. Nr. | A | $R^1$ | Y | Analyse % Ber. | Gef. | Fp. [°C] |
|---|---|---|---|---|---|---|
| 2.1 | CH | H | 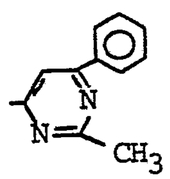 | C 63.93<br>H 5.21<br>N 12.76 | 63.8<br>5.3<br>12.7 | - |
| 2.2 | CH | H | 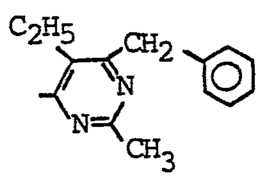 | C 65.23<br>H 5.76<br>N 12.01 | 65.0<br>5.8<br>11.8 | - |
| 2.3 | CH | H | 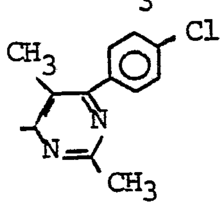 | C 61.24<br>H 5.00<br>N 11.90 | 60.1<br>5.4<br>11.3 | 150-51 |
| 2.4 | CH | H | 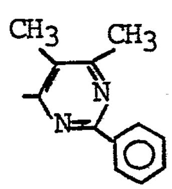 | C 64.38<br>H 5.40<br>Cl 10.56<br>N 12.51 | 64.2<br>5.6<br>11.1<br>12.3 | 76-77 |

Tabelle 2 (Fortsetzung)

| Verb. Nr. | A | $R^1$ | Y | Analyse % Ber. | Gef. | Fp. \|°C\| |
|---|---|---|---|---|---|---|
| 2.5 | CH | $CH_3$ | 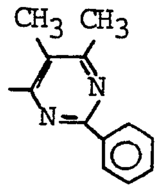 | C 65.23<br>H 5.76<br>Cl 10.13<br>N 12.01 | 65.1<br>5.7<br>10.5<br>11.7 | - |
| 2.6 | CH | H | 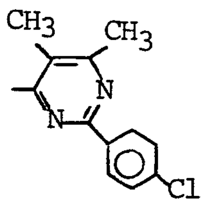 | C 61.24<br>H 5.00<br>N 11.90 | 61.5<br>5.1<br>11.7 | 158-59 |
| 2.7 | CH | H | 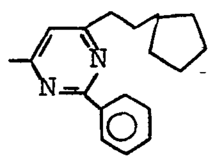 | C 66.57<br>H 6.00<br>N 11.36 | 66.3<br>6.0<br>11.3 | - |
| 2.8 | N | $CH_3$ | 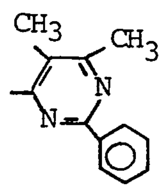 | C 63.42<br>H 5.61<br>N 13.99 | 63.1<br>5.7<br>13.6 | - |
| 2.9 | N | H | 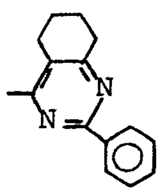 | C 63.61<br>H 5.34<br>N 14.03 | 63.6<br>5.3<br>13.9 | - |
| 2.10 | CH | H | " | C 65.42<br>H 5.49<br>Cl 10.16<br>N 12.05 | 65.1<br>5.3<br>10.6<br>11.7 | 119-20 |
| 2.11* | CH | H | 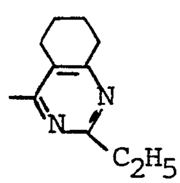 | C 62.87<br>H 5.89<br>N 12.94 | 62.0<br>5.9<br>13.5 | 142-43 |

Tabelle 2 (Fortsetzung)

| Verb. Nr. | A | $R^1$ | Y | Analyse % Ber. | Gef. | Fp. [°C] |
|---|---|---|---|---|---|---|
| 2.12 | CH | H | 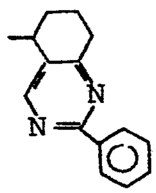 | C 65.42<br>H 5.49<br>Cl 10.16<br>N 12.05 | 65.4<br>5.3<br>10.3<br>11.9 | 96-97 |
| 2.13 | N | $CH_3$ | 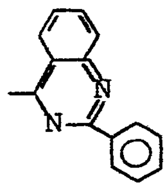 | C 64.82<br>H 5.16<br>N 13.57 | 64.4<br>5.0<br>13.2 | – |
| 2.14 | CH | H | 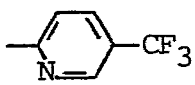 | C 56.79<br>H 4.45<br>N 11.04 | 56.3<br>4.5<br>11.3 | – |
| 2.15 | N | H | " | C 54.81<br>H 4.28<br>N 13.23 | 54.7<br>4.4<br>13.1 | 99-100 |
| 2.16 | CH | $CH_3$ | " | C 58.01<br>H 4.87<br>N 10.57 | 57.5<br>4.9<br>10.4 | – |
| 2.17 | CH | H | 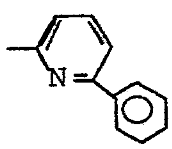 | C 65.42<br>H 5.18<br>N 10.90 | 66.2<br>5.1<br>10.9 | – |
| 2.18* | CH | H | 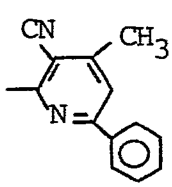 | C 65.20<br>H 5.03<br>N 12.33 | 65.1<br>5.0<br>12.0 | 127-28 |
| 2.19* | N | H | " | C 63.34<br>H 4.87<br>N 14.36 | 63.5<br>4.8<br>14.6 | 117-18 |

Tabelle 2 (Fortsetzung)

| Verb. Nr. | A | $R^1$ | Y | Analyse % Ber. | Gef. | Fp. [°C] |
|---|---|---|---|---|---|---|
| 2.20 | N | H | 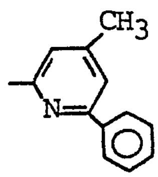 | C 63.44 | 63.0 | - |
| | | | | H 5.93 | 5.7 | |
| | | | | N 12.68 | 12.7 | |
| 2.21 | CH | H | 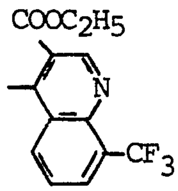 | C 58.73 | 58.5 | - |
| | | | | H 4.53 | 4.6 | |
| | | | | N 9.26 | 9.0 | |
| 2.22 | N | H | " | C 57.07 | 56.1 | - |
| | | | | H 4.39 | 4.3 | |
| | | | | N 11.09 | 10.9 | |
| 2.23 | CH | H | 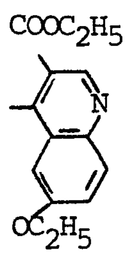 | C 62.29 | 62.0 | 118-19 |
| | | | | H 5.37 | 5.2 | |
| | | | | N 9.56 | 9.2 | |
| 2.24* | CH | H | 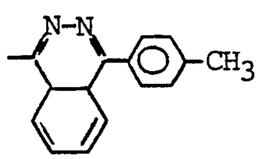 | C 66.19 | 65.0 | 254-55 |
| | | | | H 5.13 | 5.0 | |
| | | | | N 11.88 | 10.9 | |
| 2.25* | N | H | " | C 64.40 | 63.7 | 246-47 |
| | | | | H 4.98 | 5.0 | |
| | | | | N 13.84 | 13.7 | |

Beispiel 5

Zu einer Lösung von 1.62 g (5 mMol) 1-(4-Hydroxyphenyl)-4-(5-trifluormethyl-pyrid-2-yl)-piperazin in 20 ml abs. DMF gab man bei Raumtemperatur 0.58 g (5.15 mMol) Kalium-tert.-butylat, rührte 10 Min., gab dann eine Lösung von 2,04 g (5 mMol) IIb (vgl. Beispiel 3, Tab. 1) in 15 ml

abs. 1,2-Dimethoxyethan (DME) zu und rührte die Mischung 5,5 Stunden bei 92-93°C. Anschließend wurden die Lösungsmittel im Vakuum abgezogen, der Rückstand in Wasser/$CH_2Cl_2$ aufgenommen und nach Durchschütteln die Phasen getrennt. Die wäßrige Phase wurde noch zweimal mit $CH_2Cl_2$ extrahiert. Die vereinigten $CH_2Cl_2$-Auszüge wurden getrocknet, filtriert und im Vakuum eingedampft. Der verbleibende Rückstand (3,4 g) wurde an einer Kieselgel/$CH_2Cl_2$-Säule (⌀ 2,1 cm, H 30 cm) durch Elution mit $CH_2Cl_2$ und $CH_2Cl_2/C_2H_5OH$-Mischungen ($C_2H_5OH$-Gehalt: 0,1-2,0 Vol%) chromatographiert. Nach Vereinigung und Eindampfen DC-einheitlicher Fraktionen wurden 2,0 g (= 63 % Ausbeute) Cis-2-(2,4-Dichlorphenyl)-2-(imidazol-1-ylmethyl)-4-[4-(4-(5-trifluormethyl-pyrid-2-yl)-piperazin-1-yl)-phenoxymethyl]-1,3-dioxolan als hochviskoses Öl erhalten.

Beispiel 6

Nach der gleichen Arbeitsweise wie im Beispiel 5 beschrieben, gleichfalls im 5 mMol-Maßstab, wurde bei Verwendung des gleichen Piperazin-Derivats als Zwischenprodukt und bei Anwendung von IIb (vgl. Beispiel 3, Tab. 1) und bei Anwendung von 0,207 g (5.3 mMol) Natriumamid, anstelle von Kalium-tert.-butylat als Base, unter sonst gleichen Reaktionsbedingungen und bei gleicher Aufarbeitung wie im Beispiel 5 beschrieben, Cis-2-(2,4-Dichlorphenyl)-2-(imidazol-1-ylmethyl)-4-[4-(4-(5-trifluormethyl-pyrid-2-yl)-piperazin-1-yl)-phenoxymethyl]-1,3-dioxolan, 2.11 g (= 66.6 % Ausbeute), erhalten.

Beispiel 7 (Salzbildung)

Eine Lösung von 1.11 g (1,75 mMol) 2-S,(R)-(2,4-Dichlorphenyl)-2-(imidazol-1-ylmethyl)-4-R,(S)-[4-(4-(5-trifluormethyl-pyrid-2-yl)-piperazin-1-yl)-phenoxymethyl]-1,3-dioxolan (cis-Form) (vgl. Beispiel 5 und 6) in 15 ml Ethylacetat wurde mit 0,585 ml einer 6m Lösung von HCl in Ether versetzt, woraufhin kristalliner Niederschlag ausfiel.

Die Mischung wurde im Vakuum eingedampft, der verbleibende kristalline Rückstand mit 25 ml Aceton 8 Minuten aufgekocht und nach Abkühlung auf <9°C abgesaugt und getrocknet. Man erhielt 1,22 g (= 98,5 % Ausbeute an Dihydrochlorid) Cis-2-(2,4-Dichlorphenyl)-2-(imidazol-1-ylmethyl)-4-[4-(4-(5-trifluormethyl-pyrid-2-yl)-piperazin-1-yl)-phenoxymethyl]-1,3-dioxolan-dihydrochlorid; Fp. 205-06°C;
Analyse: $C_{30}H_{30}Cl_4F_3N_5O_3$ (MG 707,44),
Ber. C 50.93, H 4.27, $Cl^{\ominus}$ 10.02, N 9.90;
Gef. C 51.7 H 4.3 $Cl^{\ominus}$ 7.9 N 9.9 %.

Beispiele für das Herstellungsverfahren Variante B):

Beispiel 8

a) Zu einer Lösung von 4.64 g (12 mMol) 1-(4-Hydroxyphenyl)-4-(5,6,7,8-tetrahydro-2-phenyl-chinazolin-4-yl)-piperazin in 48 ml abs. DMF gab man bei Raumtemperatur (unter Kühlung) 0,367 g (12,24 mMol) 80 %ige Natriumhydrid-Öl-Dispersion. Nach Ende der Wasserstoffentwicklung wurde bei Raumtemperatur eine Lösung von 4,43 g (12 mMol) Cis-2-Brommethyl-2-(4-fluorphenyl)-4-methansulfonyloxymethyl-1,3-dioxolan (cis und trans bezieht sich auf die Brommethyl- und die Methansulfonyloxymethyl-Gruppe in der 2- bzw. der 4-Stellung des Dioxolanringes) in 35 ml abs. DMF zugetropft und die Mischung 4 Stunden bei 100°C gerührt. Anschließend verdampfte man das DMF im Vakuum, nahm den Rückstand in Ether/Wasser auf, trennte nach intensiver Durchmischung die Phasen und schüttelte die wäßrige Phase noch dreimal mit Ether aus. Die Etherextrakte wurden vereinigt, getrocknet und im Vakuum eingedampft. Der verbleibende Rückstand (8,0 g) wurde an einer Kieselgel S-$CH_2Cl_2$/Petrolether 1:1-Säule (ø 3,0 cm, H 41 cm) unter Elution mit $CH_2Cl_2$/Petrolether-Mischungen mit steigendem $CH_2Cl_2$-Gehalt (bis max. 75 Vol.% $CH_2Cl_2$) chromatographiert. Nach Elution von Vorzonen (Gehalt ca. 1 g) wurden DCnahezu einheitliche Fraktionen vereinigt, eingedampft und

aus Ether kristallisiert. Man erhielt auf diese Weise 6,0 g (= 76 % Ausbeute) reines Cis-2-Brommethyl-2-(4-fluorphenyl)-4-[4-(4-(5,6,7,8-tetrahydro-2-phenyl-chinazolin-4-yl)-piperazin-1-yl)-phenoxymethyl]-1,3-dioxolan, Fp. 170-71°; Analyse: $C_{35}H_{36}BrFN_4O_3$ (MG 659,62)
Ber. C 63.73, H 5.50, Br 12.12, N 8.49;
Gef. C 63,4 , H 5.5 , Br 12.6 , N 8.4 %.

b) Zu einer Lösung von 1.23 g (18.1 mMol) Imidazol in 25 ml abs. Dimethylsulfoxid gab man bei Raumtemperatur 0.54 g (18 mMol) 80 %ige Natriumhydrid -Öl-Dispersion und rührte 30 Min. bei Raumtemperatur. Anschließend setzte man 5.94 g (9.2 mMol) Cis-2-Brommethyl-2-(4-fluorphenyl)-4-[4-(4-(5,6,7,8-tetrahydro-2-phenyl-chinazolin-4-yl)-piperazin-1-yl)-phenoxymethyl]-1,3-dioxolan (unter a) hergestellt) zu und rührte 30 Stunden unter Stickstoffatmosphäre bei 130°C. Das Dimethylsulfoxid (DMSO) wurde im Ölpumpenvakuum am Rotationsverdampfer abdestilliert. Der verbleibende Rückstand wurde in $CH_2Cl_2$/Waser aufgenommen. Nach Durchmischung und Trennung der Phasen wurde die wäßrige Lösung dreimal mit $CH_2Cl_2$ extrahiert. Die vereinigten $CH_2Cl_2$-Extrakte wurden im Vakuum eingedampft. Der verbleibende Rückstand (6,5 g) wurde an einer Kieselgel S-$CH_2Cl_2$/Petrolether 1:1-Säule (⌀ 2,6 cm, Höhe 40 cm) unter Elution mit $CH_2Cl_2$/Petrolether 1:1; 2:1-4:1; $CH_2Cl_2$ und $CH_2Cl_2$/$C_2H_5OH$-Mischungen mit steigendem $C_2H_5OH$-Gehalt (bis max. 1.6 Vol.% $C_2H_5OH$) chromatographiert. Nach Vereinigen und Eindampfen der DC-einheitlichen Fraktionen wurden 1.67 g (= 28.7 % Ausbeute) reines Cis-2-(4-Fluorphenyl)-2-(imidazol-1-ylmethyl)-4-[4-(4-(5,6,7,8-tetrahydro-2-phenyl-chinazolin-4-yl)-piperazin-1-yl)phenoxymethyl]-1,3-dioxolan, Fp. 161-62°C, erhalten; Analyse: $C_{38}H_{39}FN_6O_3$ (MG 646.78)
Ber. C 70.57, H 6.08, F 2.94, N 12.99;
Gef. C 69.9 , H 6.0 , F 2.7 , N 12.8 %.

Beispiel 9

a) Eine Mischung von 3,605 g (10 mMol) 1-(4-Hydroxyphenyl)-4-(2-methyl-4-(4-tolyl)-pyrimidin-6-yl)-piperazin, 80 ml Toluol, 4.20 g (10 mMol) 2-Brommethyl-2-(2,4-dichlorphenyl)-4-methansulfonyloxymethyl-1,3-dioxolan (cis/trans-Mischung), 0.65 g Tetrabutylammoniumbromid und 13,5 ml 50 %ige Natronlauge wurde 4 Stunden bei 55°C intensiv gerührt. Anschließend trennte man bei Raumtemperatur die Phasen, schüttelte die Natronlauge dreimal mit Ether aus und vereinigte Toluol- und Etherphasen. Diese wurden dreimal mit Wasser ausgewaschen, getrocknet, filtriert und im Vakuum eingedampft. Der verbleibende Rückstand (7,4 g) wurde an einer Kieselgel S/$CH_2Cl_2$-Säule (ø 2,6 cm, H 11 cm) unter Elution mit $CH_2Cl_2$ chromatographiert.
Nach Elution wurden die DC-einheitlichen Fraktionen vereinigt und im Vakuum eingedampft. Man erhielt 6,27 g (= 91,6 % Ausbeute) 2-Brommethyl-2-(2,4-dichlorphenyl)-4-[4-(4-(2-methyl-4-(4-tolyl)-pyrimidin-6-yl)-piperazin-1-yl)-phenoxymethyl]-1,3-dioxolan (Cis/trans-Mischung) als hochviskoses Öl; Analyse: $C_{33}H_{33}BrCl_2N_4O_3$ (MG 684.49)
Ber. C 57.91, H 4.86, Br 11.68, Cl 10.36, N 8.19;
Gef. C 57.8 H 4.8 Br 11.8 Cl 10.5 N 8.0 %

b) Wie im Beispiel 8b beschrieben wurden 6,21 g (9.1 mMol) 2-Brommethyl-2-(2,4-dichlorphenyl)-4-[4-(4-(2-methyl-4-(4-tolyl)-pyrimidin-6-yl)-piperazin-1-yl)-phenoxymethyl]-1,3-dioxolan (cis/trans-Diastereomerenmischung) mit 1,244g (18.3 mMol) Imidazol und 0,55 g (18,2 mMol) 80 %iger Natriumhydrid-Öl-Dispersion in 26 ml abs. Dimethylsulfoxid umgesetzt. Nach 26 Stunden Rühren bei 130°C wurde das Dimethylsulfoxid (DMSO) im Ölpumpenvakuum abdestilliert. Der verbleibende Rückstand wurde in $CH_2Cl_2$/Wasser aufgenommen. Nach Durchmischung und anschließender Trennung der Phasen wurde die wäßrige Lösung dreimal mit $CH_2Cl_2$ extrahiert. Die vereinigten $CH_2Cl_2$-Extrakte wurden im Vakuum eingedampft. Der verbleibende Rückstand (4.30 g

wurde an einer Kieselgel S/$CH_2Cl_2$-Säule (ø 2,0 cm, H 20cm) unter Elution mit $CH_2Cl_2$ und $CH_2Cl_2/C_2H_5OH$-Mischungen (0,5-3,0 Vol% $C_2H_5OH$) chromatographiert. Die die Diastereomeren-Racemate enthaltenden Fraktionen (Überprüfung mittels DC) wurden vereinigt und im Vakuum eingedampft. Auf diese Weise erhielt man 3.12 g (= 51 % Ausbeute) 2-(2,4-Dichlorphenyl)-2-(imidazol-1-ylmethyl)-4-[4-(4-(2-methyl-4-(4-tolyl)-pyrimidin-6-yl)-piperazin-1-yl)-phenoxymethyl]-1,3-dioxolan (cis/trans-Diastereomeren-Mischung) als zähflüssiges Öl; Analyse: $C_{36}H_{36}Cl_2N_6O_3$ (MG 671.65)
Ber. C 64.38, H 5.40, N 12.51;
Gef. C 62.8 , H 5.6 , N 11.8 %.

Beispiel 10
Nach der gleichen Arbeitsweise wie im Beispiel 8a beschrieben wurden, ausgehend von 20 mMol 1-(4-Hydroxyphenyl)-4-(5-trifluormethyl-pyrid-2-yl)-piperazin, der entsprechenden Menge NaH und 20 mMol 2-Brommethyl-2-(2,4-dichlorphenyl)-4-methansulfonyloxymethyl-1,3-dioxolan (cis/trans-Mischung), 8.92 g (= 69 % d.Th.) 2-Brommethyl-2-(2,4-dichlorphenyl)-4-[4-(4-(5-trifluormethyl-pyrid-2-yl)-piperazin-1-yl)phenoxymethyl]-1,3-dioxolan (cis/trans-Mischung) als hochviskoses Öl erhalten; Analyse: $C_{27}H_{25}BrCl_2F_3O_3$ (MG 647.35)
Ber. C 50.10, H 3.89, F 8.81, N 6.49 ;
Gef. C 49.2 , H 3.7 , F 8.2 , N 6.6 %.

Beispiel 11
Zu einer Suspension von 0.49 g (16,3 mMol) 80 %iger NaH Öl-Dispersion in 15 ml abs. Dimethylsulfoxid (DMSO) tropfte man bei Raumtemperatur eine Lösung von 1.03 g (14,9 mMol) 1,2,4-Triazol in 7 ml abs. DMSO, rührte 30 Minuten bei Raumtemperatur nach und gab anschließend eine Lösung von 6.475 g (10 mMol) gemäß Beispiel 10 hergestelltes 2-Brommethyl-2-(2,4-dichlorphenyl)-4-[4-(4-(5-trifluormethyl-pyrid-2-yl)-piperazin-1-yl)-phenoxymethyl]-1,3-dioxolan (cis/trans-Mischung) in 7 ml abs. DMSO zu und rührte 28

Stunden bei 130°C unter Stickstoffatmosphäre. Nach Abkühlung wurde die Reaktionsmischung in 140 ml Wasser eingerührt und die entstandene Mischung mehrmals mit $CH_2Cl_2$ extrahiert. Die vereinigten $CH_2Cl_2$-Extrakte wurden getrocknet, filtriert und im Vakuum eingedampft. Der Rückstand (7.0 g) wurde wie im Beispiel 9b beschrieben an einer Kieselgel S-$CH_2Cl_2$-Säule (ø 2,6 cm, H 40.0 cm) durch Chromatographie gereinigt. Aus den laut DC gleichen Fraktionen wurden 1,72g (= 27,0 % Ausbeute) nahezu reines 2-(2,4-Dichlorphenyl)-2-(1,2,4-triazol-1-ylmethyl)-4-[4-(4-(5-trifluormethyl-pyrid-2-yl)-piperazin-1-yl)-phenoxymethyl]-1,3-dioxolan (cis/trans-Diastereomeren-Mischung) als hochviskoses Öl erhalten; Analyse: $C_{29}H_{27}Cl_2F_3N_6O_3$ (MG 635,50)

Ber. C 54.81, H 4.28, F 8.97, N 13.23;

Gef. C 54.1 , H 4.4 , F 8.2 , N 12.8 %.

Beispiele für das Herstellungsverfahren Variante D)

Beispiel 12

Eine Lösung von 3,67 g (7,5 mMol) 2-S,(R)-(2,4-Dichlorphenyl)-2-(imidazol-1-ylmethyl)-4-R,(S)-(4-piperazinophenoxymethyl)-1,3-dioxolan(cis-Form) und 1,40 g (7,7 mMol) 2-Chlor-5-trifluormethyl-pyridin in 30 ml abs. DMF wurde auf 80°C unter Stickstoffatmosphäre erwärmt und unter Rühren nach 10 Minuten mit 173 mg pulverisiertem Kaliumkarbonat versetzt. Nach weiteren 25 Minuten gab man weitere 173 mg pulverisiertes $K_2CO_3$ und nach weiteren 60 Minuten eine 3. Portion von 173 mg pulverisiertem $K_2CO_3$ zu (insgesamt 519 mg (3,75 mMol) $K_2CO_3$). Anschließend rührte man 9 Stunden bei 80°C nach, destillierte das DMF im Ölpumpenvakuum am Rotationsverdampfer ab und nahm den Rückstand in Wasser/$CH_2Cl_2$ auf. Nach Durchmischung und Trennung der Phasen wurde die wäßrige Lösung dreimal mit $CH_2Cl_2$ extrahiert. Die vereinigten $CH_2Cl_2$-Extrakte wurden getrocknet,

filtriert und im Vakuum eingedampft. Der Rückstand wurde durch Säulenchromatographie an Kieselgel S/$CH_2Cl_2$ (ø 2,6 cm, H 29 cm) unter Elution mit $CH_2Cl_2$ und $CH_2Cl_2$/$C_2H_5OH$-Mischungen mit steigendem $C_2H_5OH$-Gehalt (bis max. 4 Vol.% $C_2H_5OH$) gereinigt. Man erhielt 2,47 g (= 52 % Ausbeute) reines Cis-2-(2,4-Dichlorphenyl)-2-(imidazol-1-ylmethyl)-4-[4-(4-(5-trifluormethyl-pyrid-2-yl)-piperazin-1-yl)-phenoxymethyl]-1,3-dioxolan als zähes Öl;

Analyse: $C_{30}H_{28}Cl_2F_3N_5O_3$

Ber. C 56.79, H 4.45, N 11.04;

Gef. C 56.4 , H 4.3 , N 11.0 %.

Beispiel 13

Nach der gleichen Arbeitsweise wie im Beispiel 12 beschrieben, ausgehend von IXa oder IXb (vgl. Tabelle 3) und jeweils der entsprechenden Verbindung der Formel Xa, wurden die in der Tabelle 3 aufgeführten Verbindungen der Formel I nach Verfahrensvariante D) hergestellt. Bei Verwendung von 4-Chlorpyrimidinen erfolgte die Zugabe von $K_2CO_3$ und das Nachrühren (5-7 Stunden) bei 90°C und bei Verwendung von 4-Chlor-chinolinen erfolgten diese Maßnahmen bei 80°C (Nachrührzeit 4-5 Stunden).

Tabelle 3

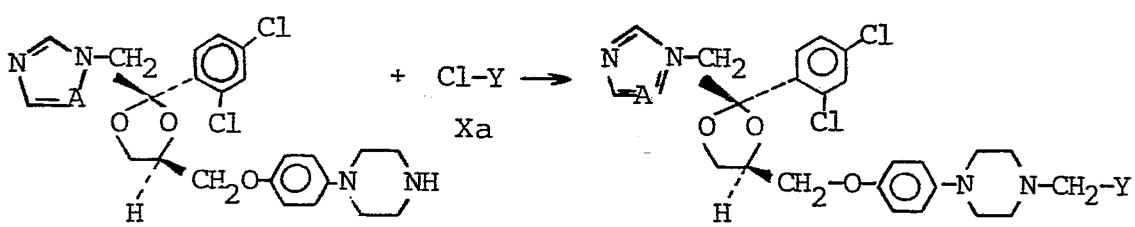


IX a (A = CH)

IX b (A = N)

cis

cis I (Ar= 2,4-$Cl_2$-$C_6H_3$-, q=0)

| Verb. Nr. | A | Y | Analyse Ber. | Gef. | Fp. [°C] |
|---|---|---|---|---|---|
| 3.1 | CH | 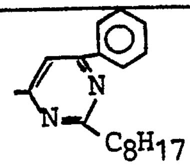 | C 66.74<br>H 6.40<br>N 11.12 | 66.2<br>6.1<br>10.4 | - |
| 3.2 | N | 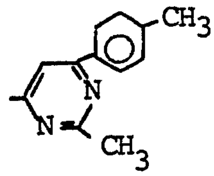 | C 62.50<br>H 5.25<br>N 14.58 | 62.1<br>5.2<br>14.3 | 122-23 |
| 3.3 | N | 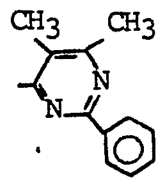 | C 62.50<br>H 5.25<br>N 14.58 | 62.0<br>5.1<br>14.2 | - |
| 3.4 | CH | 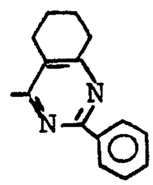 | C 65.42<br>H 5.49<br>N 12.05 | 65.0<br>5.2<br>12.2 | 119-20 |
| 3.5 | CH | 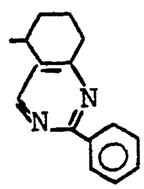 | C 65.42<br>H 5.49<br>N 12.05 | -<br>-<br>- | 96-97 |
| 3.6 | CH | 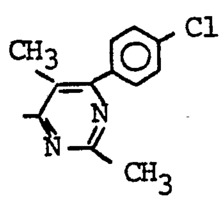 | C 61.24<br>H 5.00<br>N 11.90 | 60.9<br>4.9<br>11.6 | 151-52 |
| 3.7 | N | 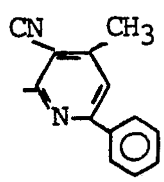 | C 63.34<br>H 4.87<br>N 14.36 | 63.2<br>4.8<br>14.2 | 117-18 |
| 3.8 | CH | 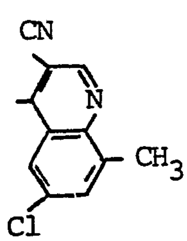 | C 60.92<br>H 4.53<br>N 12.18 | 60.1<br>4.2<br>11.8 | 207-09 |

Beispiel 14

Eine Mischung von 3,42 g ( 7 mMol) Cis-2-(2,4-Dichlorphenyl)-2-(imidazol-1-ylmethyl)-4-(4-piperazino-phenoxymethyl)-1,3-dioxolan, 1,69 g ( 7 mMol) 4-Chlor-2-phenyl-chinazolin, 0,45 g Tetrabutylammoniumbromid, 55 ml Toluol und 9 ml 50 %ige Natronlauge wurde 5 Stunden bei 70°C intensiv gerührt. Danach trennte man bei Raumtemperatur die Phasen, schüttelte die konzentrierte NaOH zweimal mit Ether aus, vereinigte Toluol- und Etherphasen und schüttelte diese dreimal mit Wasser aus. Die Toluol-Ether-Lösung wurde getrocknet, filtriert und im Vakuum eingedampft. Der verbleibende Rückstand (4,95 g) wurde an einer Kieselgel S/ $CH_2Cl_2$-Säule (⌀ 2,6 cm, H 30 cm) wie im Beispiel 1 beschrieben chromatographiert. Man erhielt nach Vereinigen und Eindampfen DC-einheitlicher Fraktionen 2,49 g (= 52 % Ausbeute) Cis-2-(2,4-Dichlorphenyl)-2-(imidazol-1-ylmethyl)-4-[4-(4-(2-phenyl-chinazolin-4-yl)-piperazin-1-yl)-phenoxymethyl]-1,3-dioxolan als hochviskoses Öl;

Analyse: $C_{38}H_{34}Cl_2N_6O_3$ (MG 693.65)
Ber. C 65.80, H 4.94, Cl 10.22, N 12.12;
Gef. C 64.8 H 4.8 Cl 10.6 N 11.8 %.

Beispiele für die Herstellung von Verbindungen der Formel IIIa

Beispiel 15

Eine Lösung von 1.89 g (10.6 mMol) 1-(4-Hydroxyphenyl)-piperazin und 2.19 g (10 mMol) 4-Chlor-5,6-dimethyl-2-phenyl-pyrimidin in 30 ml abs. N,N-Dimethylformamid (DMF) wurde auf 80°C erwärmt und unter Rühren bei 80°C nach 10 Minuten, nach weiteren 25 Minuten und nach weiteren 60 Minuten mit jeweils 234 mg pulverisiertem $K_2CO_3$ unter Stickstoffatmosphäre versetzt (insgesamt Zusatz von 702 mg (5.08 mMol) $K_2CO_3$). Man rührte 6 Stunden bei 90°C nach,

destillierte im Ölpumpenvakuum am Rotationsverdampfer das DMF weitgehend ab und nahm den verbleibenden Rückstand in $CH_2Cl_2$/Wasser auf und stellte mit verdünnter Salzsäure den pH auf 7-8. Nach Durchmischung und Trennung der Phasen wurde die wäßrige noch zweimal mit $CH_2Cl_2$ extrahiert. Die vereinigten $CH_2Cl_2$-Extrakte wurden getrocknet, filtriert und im Vakuum eingedampft. Der kristalline Rückstand (3,6g) wurde an einer Kieselgel S/$CH_2Cl_2$-Säule (ø 2,0 cm, H 38 cm) durch Elution mit $CH_2Cl_2$ und $CH_2Cl_2$/$C_2H_5OH$-Mischungen mit steigendem $C_2H_5OH$-Gehalt (bis max. 10 Vol.% $C_2H_5OH$) chromatographiert. Aufgezogen wurde die Substanz auf die Säule mit 60 ml einer $CH_2Cl_2$/Tetrahydrofuran 2:1-Mischung. Die eluierte, nicht ganz reine, kristalline Substanz wurde durch Auskochen mit wenig $CH_2Cl_2$ und Absaugen gereinigt. Man erhielt so 2,22 g reines 1-(4-Hydroxyphenyl)-4-(5,6-dimethyl-2-phenyl-pyrimidin-4-yl)-piperazin, Fp. 202-03°C, (= 61,5 % Ausbeute) Analyse: $C_{22}H_{24}N_4O$ (MG 360.47),
Ber. C 73.31, H 6.71, N 15.54,
Gef. C 73.2 , H 6.6 , N 15.6 %.

Beispiel 16

Eine Mischung von 4,42 g (12 mMol) 1-(4-Hydroxy-3,5-dimethylphenyl)-piperazin-dihydrobromid, 2.84 g (13 mMol) 4-Chlor-5,6-dimethyl-2-phenyl-pyrimidin, 1,66 g (12 mMol) pulverisiertem $K_2CO_3$ und 38 ml abs. DMF wurde auf 90°C erwärmt und unter Rühren bei 90°C nach 10 Minuten, nach weiteren 25 Minuten und nach weiteren 60 Minuten mit jeweils 277 mg pulverisiertem $K_2CO_3$ (insgesamt 6 mMol $K_2CO_3$) versetzt und 6 Stunden bei 95°C nachgerührt. Anschließend destillierte man das DMF im Vakuum ab, nahm den Rückstand in $CH_2CL_2$/Wasser auf, trennte nach Durchmischung die Phasen und extrahierte die wäßrige noch dreimal mit $CH_2Cl_2$. Die vereinigten $CH_2Cl_2$-Extrakte wurden getrocknet, filtriert und im Vakuum eingedampft. Den kristallinen Rückstand (5,2 g) kochte man mit 15 ml Methanol auf und saugte nach Abkühlung im Eisbad das Kristallisat ab. Man erhielt auf

diese Weise 3,54 g (= 76 % Ausbeute) reines 1-(4-Hydroxy-3,5-dimethyl-phenyl)-4-(5,6-dimethyl-2-phenylpyrimidin-4-yl)-piperazin, Fp. 188-89°C, Analyse $C_{24}H_{28}N_4O$ (MG 388.52)
Ber. C 74.20, H 7.26, N 14.4;
Gef. C 74.0 , H 7.3 , N 14.3 %.

Beispiel 17

Gemäß der in den Beispielen 15 und 16 beschriebenen Arbeitsmethode wurden ausgehend von einer Verbindung der Formel XIIIa und jeweils des entsprechenden 4-Chlorpyrimidins der Formel Xa (vgl. Tab. 4) die in der Tabelle 4 aufgeführten Verbindungen der Formel IIIa hergestellt. Sofern der nach Abdestillieren des DMF verbliebene Rückstand kristallin war bzw. beim Aufnehmen in Wasser kristallisierte und durch Umkristallisieren (bevorzugt aus Methanol oder Acetonitril) zu reinigen war, wurde die betreffende Verbindung der Formel IIIa auf diese Weise rein hergestellt. Andernfalls wurden die Verbindungen IIIa durch Säulenchromatographie gemäß der im Beispiel 15 beschriebenen Arbeitsweise in reiner Form gewonnen. Diese Fälle sind in der Tabelle 4 mit (*) gekennzeichnet.

Tabelle 4

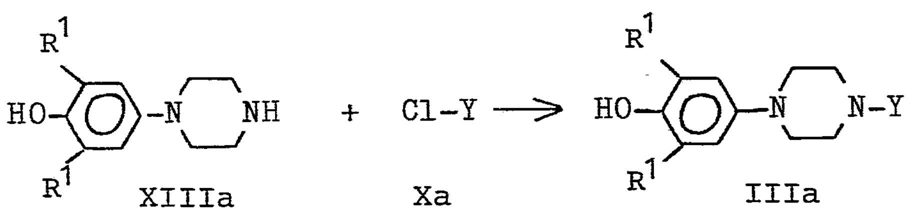

XIIIa Xa IIIa

| Verb. Nr. | $R^1$ | Y | Ausbeute [%] | Fp [°C] | Summenformel | Analyse % Ber. | Gef. |
|---|---|---|---|---|---|---|---|
| 4.1 | H | 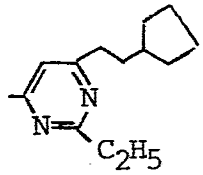 | 67 | 187-88 | $C_{23}H_{32}N_4O$ | C 72.60<br>H 8.47<br>N 14.72 | 72.5<br>8.4<br>14.5 |
| 4.2*) | $CH_3$ | " | 85 | 134-35 | $C_{25}H_{36}N_4O$ | C 73.49<br>H 8.88<br>N 13.71 | 73.2<br>9.2<br>13.5 |

Tabelle 4 (Fortsetzung)

| Verb. Nr. | $R^1$ | Y | Ausbeute [%] | Fp [°C] | Summenformel | Analyse % Ber. | Gef. |
|---|---|---|---|---|---|---|---|
| 4.3 | H | 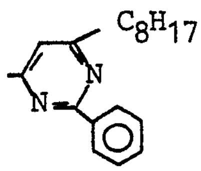 | 58 | 121-22 | $C_{28}H_{36}N_4O$ | C 75.64<br>H 8.16<br>N 12.60 | 75.3<br>8.0<br>12.7 |
| 4.4 | H | 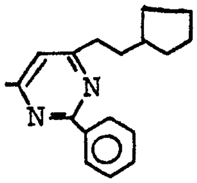 | 61 | 147-48 | $C_{27}H_{32}N_4O$ | C 75.67<br>H 7.53<br>N 13.07 | 75.6<br>7.6<br>12.9 |
| 4.5 | H | 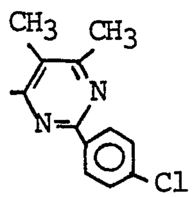 | 50 | 205-06 | $C_{22}H_{23}ClN_4O$ | C 66.91<br>H 5.87<br>N 14.19 | 66.8<br>5.8<br>14.0 |
| 4.6 | H | 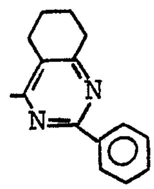 | 82 | 227-28 | $C_{24}H_{26}N_4O$ | C 74.58<br>H 6.78<br>N 14.50 | 74.3<br>6.8<br>14.4 |
| 4.7 | | 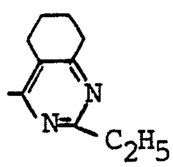 | 82 | 187-88 | $C_{20}H_{26}N_4O$ | C 70.97<br>H 7.74<br>N 16.55 | 70.7<br>7.8<br>16.4 |
| 4.8 | H | 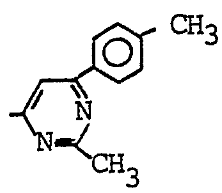 | 89 | 232-33 | $C_{22}H_{24}N_4O$ | C 73.31<br>H 6.71<br>N 15.54 | 73.2<br>6.7<br>15.4 |
| 4.9 | H | 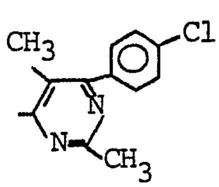 | 87 | 252-53 | $C_{22}H_{23}ClN_4O$ | C 66.91<br>H 5.87<br>N 14.19 | 66.7<br>5.8<br>14.1 |
| 4.10*) | H | 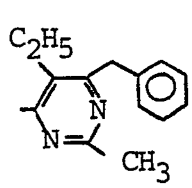 | 38 | 148-49 | $C_{24}H_{28}N_4O$ | C 74.20<br>H 7.26<br>N 14.42 | 73.2<br>7.0<br>14.0 |

Tabelle 4 (Fortsetzung)

| Verb. Nr. | $R^1$ | Y | Ausbeute [%] | Fp [°C] | Summenformel | Analyse % Ber. | Gef. |
|---|---|---|---|---|---|---|---|
| 4.11*) | $CH_3$ | 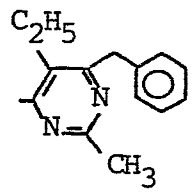 | 42 | - | $C_{26}H_{32}N_4O$ | C 74.96<br>H 7.74<br>N 13.45 | 74.6<br>7.6<br>13.4 |
| 4.12*) | H | 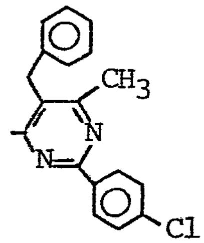 | 64 | 171-72 | $C_{28}H_{27}ClN_4O$ | C 71.40<br>H 5.78<br>N 11.90 | 71.1<br>5.7<br>11.6 |
| 4.13 | H | 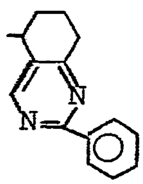 | 53 | 240-42 | $C_{24}H_{26}N_4O$ | C 74.58<br>H 6.78<br>N 14.50 | 74.4<br>6.7<br>14.3 |
| 4.14 | H | 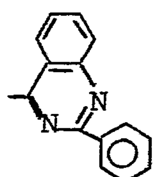 | 86 | 191-92 | $C_{24}H_{22}N_4O$ | C 75.37<br>H 5.80<br>N 14.65 | 75.4<br>5.6<br>14.6 |
| 4.15*) | H | 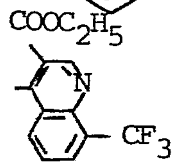 | 72 | 168-69 | $C_{23}H_{22}F_3N_3O_3$ | C 61.99<br>H 4.99<br>N 9.44 | 61.5<br>4.7<br>9.2 |
| 4.16#) | H | 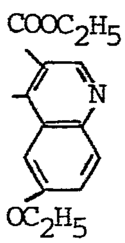 | 22 | 196-97 | $C_{24}H_{27}N_3O_4$ | C 68.39<br>H 6.46<br>N 9.97 | 67.0<br>6.4<br>10.2 |
| 4.17 | H | 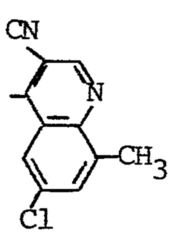 | 89 | 226-27 | $C_{21}H_{19}ClN_4O$ | C 66.57<br>H 5.06<br>N 14.79 | 66.5<br>5.1<br>14.5 |

#) 2,5 Stunden 80°C Nachrührzeit

Beispiel 18

Eine Mischung von 5.11 g (15 mMol) 1-(4-Hydroxyphenyl)-piperazin-dihydrobromid, 2,48 g (15 mMol) 1-Chlorphthalazin, 2,08 g (15 mMol) pulverisiertem $K_2CO_3$ und 55 ml abs. DMF wurde auf 85°C erwärmt und unter Rühren bei 85°C nach 10 Minuten, nach weiteren 25 Minuten und nach weiteren 60 Minuten mit jeweils 346 mg pulverisiertem $K_2CO_3$ (insgesamt 1,038 g, 7,51 mMol $K_2CO_3$) versetzt und 2 Stunden bei 90°C und 3 Stunden bei 105°C nachgerührt. Danach destillierte man das DMF im Vakuum ab, nahm den Rückstand in $CH_2Cl_2$/Wasser auf, trennte nach Durchmischung die Phasen und extrahierte die wäßrige Lösung dreimal mit $CH_2Cl_2$. Die vereinigten $CH_2Cl_2$-Extrakte wurden getrocknet, filtriert und im Vakuum eingedampft. Der verbleibende Rückstand (4,75 g) wurde an einer Kieselgel S/$CH_2Cl_2$-Säule (∅ 2,6 cm, H 43 cm) durch Elution mit $CH_2Cl_2$-$C_2H_5OH$-Mischungen mit steigendem $C_2H_5OH$-Gehalt (1-10 Vol.%) chromatographiert. Die Fraktionen, in denen laut DC die gesuchte Verbindung angereichert war, wurden zusammgengefaßt und eingedampft. Der anfallende kristalline Rückstand (2.04 g) wurde mit 15 ml $CH_2Cl_2$ kurz aufgekocht, im Eisbad abgekühlt und abgesaugt. Man erhielt so 0,73 g reines 1-(4-Hydroxyphenyl)-4-(1-phthalazinyl)-piperazin, Fp. 244-45°C. Die Mutterlauge wurde nochmals an einer Kieselgel/$CH_2Cl_2$-Säule (∅ 2,0 cm, H 22 cm) analog der ersten Säulenchromatographie chromatographisch gereinigt. Das dabei anfallende angereicherte Produkt wurde ebenso wie der erste Teil mit 7 ml $CH_2Cl_2$ kurz aufgekocht und im gekühlten Zustand abgesaugt. Hierbei erhält man weitere 0,28 g reine Verbindung, Fp.244-45°C. Die Ausbeute betrug insgesamt 1.01 g = 22 % d.Th.;

Analyse: $C_{18}H_{18}N_4O$ (MG 306.37)

Ber. C 70.57, H 5.92, N 18.29;

Gef. C 69.8 , H 5.9 , N 18.2 %.

Beispiel 19

Nach der gleichen Arbeitsweise wie im Beispiel 18 beschrieben wurden 35 mMol 1-(4-Hydroxyphenyl)-piperazin-dihydro-

bromid, 35 mMol 1-Chlor-4-(4-tolyl)-phthalazin und 52,5 mMol $K_2CO_3$ in 110 ml abs. N,N-Dimethylformamid (DMF) umgesetzt. Die Nachrührzeit betrug 2 Stunden bei 90°C und 7 Stunden bei 105°C. Danach wurde das DMF im Vakuum abdestilliert. Der verbleibende Rückstand wurde in $CH_2Cl_2$/Wasser aufgenommen. Nach Durchmischung trennte man die Phasen, extrahierte die wäßrige Lösung noch dreimal mit $CH_2Cl_2$, vereinigte die $CH_2Cl_2$-Extrakte und dampfte diese nach dem Trocknen mit $MgSO_4$ im Vakuum ein. Den Rückstand vermischte man mit 70 ml $CH_2Cl_2$ und saugte die dabei kristallin anfallende Substanz ab. Bei diesem Feststoff (1,7 g) handelt es sich um 1,2-Dihydro-4-(4-tolyl)-phthalazin-1-on, ein Nebenprodukt. Das Filtrat wurde auf 40-50 ml Volumen eingeengt, woraufhin weitere Substanz auskristallisierte, die abgesaugt wurde. Dieser Anteil (4,65 g) wurde mit 15 ml Methanol kurz aufgekocht und nach Abkühlung ( $<$10°C) abgesaugt und getrocknet. Man erhielt an dieser Stelle 2,72 g reines 1-(4-Hydroxyphenyl)-4-[4-(4-tolyl)-phthalazin-1-yl]-piperazin, Fp. 265-66°C, Analyse: $C_{25}H_{24}N_4O$ (MG 396,50)

Ber. C 75.73, H 6.10, N 14.13;

Gef. C 76.0 , H 6.2 , N 14.2 %.

Die Mutterlaugen (Filtrate) wurden zusammengefaßt, im Vakuum eingedampft und an einer Kieselgel/$CH_2Cl_2$-Säule (ø 4,2 cm, H 38 cm) unter Elution mit $CH_2Cl_2$ und $CH_2Cl_2$/$C_2H_5OH$-Mischungen (0,5-10,0 Vol.% $C_2H_5OH$) chromatographiert. Man erhielt hierbei weitere 2,9 g angereicherte Substanz, die nach Aufkochen mit 8 ml Methanol, Absaugen im gekühlten Zustand und Trocknen 2,0 g weitere reine Substanz ergaben. Die Ausbeute betrug 4,72 g (= 34 % d.Th.) 1-(4-Hydroxyphenyl)-4-[4-(4-tolyl)-phthalazin-1-yl]-piperazin.

Beispiel 20

Eine Mischung von 6,82 g (20 mMol) 1-(4-Hydroxyphenyl)-piperazindihydrobromid, 3,36 g (18,5 mMol) 2-Chlor-5-trifluormethyl-pyridin, 2,91 g (21 mMol) pulverisiertem $K_2CO_3$

und 76 ml abs. DMF wurde auf 80°C erwärmt und unter Rühren bei 80°C nach 10 Minuten, nach weiteren 25 Minuten und nach weiteren 60 Minuten mit jeweils 460 mg pulverisiertem $K_2CO_3$ (insgesamt 1,38 g) 10 mMol) $K_2CO_3$) versetzt und 9 Stunden bei 80°C nachgerührt. Nach Abdestillieren des DMF im Vakuum wurde der Rückstand in $CH_2Cl_2$/Wasser aufgenommen, nach Durchmischung die Phasen getrennt und die wäßrige Lösung dreimal mit $CH_2Cl_2$ extrahiert. Die vereinigten $CH_2Cl_2$-Extrakte wurden getrocknet, filtriert und im Vakuum eingedampft. Der Rückstand (6 g) löste man kochend in Methanol. Die gebildete trübe Lösung wurde über Kieselgur abgesaugt, auf die Hälfte eingeengt und im Eisbad gekühlt. Das ausfallende Kristallisat wurde abgesaugt und getrocknet. Das Filtrat schied nach weiterem Einengen und Kühlen weitere kristalline Substanz ab, die abgesaugt und aus wenig Methanol umkristallisiert wurde. Man erhielt auf diese Weise 3,43 g (= 57,4 % Ausbeute) 1-(4-Hydroxyphenyl)-4-(5-trifluormethyl-pyrid-2-yl)-piperazin, Fp. 178-79°C,
Analyse: $C_{16}H_{16}F_3N_3O$ (MG 323,33)
Ber. C 59.44, H 4.99, N 13.0;
Gef. C 59.0 , H 5.2 , N 13.1 %.

Beispiel 21

Nach der gleichen Arbeitsweise wie im Beispiel 20 beschrieben, wurden 2,21 g (6 mMol) 1-(4-Hydroxy-3,5-dimethylphenyl)-piperazin-dihydrobromid, 1,14 g (6.25 mMol) 2-Chlor-5-trifluormethyl-pyridin und 1,245 g (9 mMol) $K_2CO_3$ in 27 ml abs. DMF umgesetzt, Die Nachrührzeit betrug 3,5 Stunden bei 90°C. Nach Verdampfen des DMF wurde der Rückstand mit Wasser vermischt und die dabei entstandene verölte kristalline Masse abgesaugt, in Methanol gelöst und mit Aktivkohle geklärt. Nach der Filtration wurde zum Filtrat etwas Wasser zugesetzt, wobei kristalliner Niederschlag ausfiel. Dieser wurde abgesaugt (Anfall 1,63 g) und an einer Kieselgel/$CH_2Cl_2$-Säule (ø 2,0 cm, H 22 cm) wie im Beispiel 15 beschrieben chromatographiert. Man erhielt

auf diese Weise 1,24 g (= 59 % Ausbeute) 1-(4-Hydroxy-3,5-dimethyl-phenyl)-4-(5-trifluormethyl-pyrid-2-yl)-piperazin, Fp. 143-44°C, Analyse: $C_{18}H_{20}F_3N_3O$ (MG 351.38)
Ber. C 61.53, H 5.74, N 11.96;
Gef. C 61.3 , H 5.6 , H 12.1 %.

Beispiel 22

Gemäß der im Beispiel 20 beschriebenen Arbeitsweise wurden 12,26 g (36 mMol) 1-(4-Hydroxyphenyl)-piperazin-dihydrobromid, 8,24 g (36,1 mMol) 2-Chlor-3-cyano-4-methyl-6-phenyl-pyridin und 7,46 g (54 mMol) pulverisiertes $K_2CO_3$ in 135 ml abs. DMF bei 90°C umgesetzt. Die Nachrührzeit betrug 10 Stunden bei 110°C. Nach Abdestillieren des DMF wurde der Rückstand in $CH_2Cl_2$/Wasser aufgenommen. Nach Durchmischung, Trennung der Phasen und Extraktion der wäßrigen Lösung wurden die $CH_2Cl_2$-Extrakte vereinigt, getrocknet und eingedampft. Der kristalline Rückstand (14,6 g) wurde dreimal wie folgt umkristallisiert. Man löste die Substanz siedend in einer $CH_2Cl_2$/$CH_3OH$-1:1-Mischung und destillierte dann das $CH_2Cl_2$ großteils ab. Aus der erhaltenen $CH_3OH$-Lösung kristallisierte Produkt aus, das abgesaugt und erneut auf diese Weise umkristallisiert wurde. Aus den Mutterlaugen wurde weitere nicht reine Substanz durch Einengen etc. isoliert, die abschließend, ebenso wie vorstehend beschrieben, nochmals umkristallisiert wurde. Auf diese Weise wurden 6,82 g (= 51 % Ausbeute) 1-(4-Hydroxyphenyl)-4-(3-cyano-4-methyl-6-phenyl-pyrid-2-yl)-piperazin, Fp. 207-08°C, erhalten;
Analyse: $C_{23}H_{22}N_4O$ (MG 370,46)
Ber. C 74.57, H 5.99, N 15.12;
Gef. C 73.7 , H 5.9 , N 14.9 %.

Beispiel 23

Eine Mischung aus 12.77 g (37,5 mMol) 1-(4-Hydroxyphenyl)-piperazin-dihydrobromid, 7,13 g (37,6 mMol) 2-Chlor-6-phenyl-pyridin, 8 g (58 mMol) $K_2CO_3$ und 145 ml abs. DMF

wurdeunter Rühren 24 Stunden am Rückfluß gekocht. Danach wurde das DMF im Vakuum abdestilliert. Den Rückstand versetzte man mit $CH_2Cl_2$/Wasser, schüttelte durch, trennte die Phasen und extrahierte die wäßrige Lösung mehrmals mit $CH_2Cl_2$. Die vereinigten $CH_2Cl_2$-Extrakte dampfte man nach dem Trocknen und Filtrieren ein. Den Rückstand nahm man in Ethylacetat auf, woraufhin kristalliner Niederschlag ausfiel, der abgesaugt wurde. Bei diesem Feststoff (4,89 g) handelt es sich um ein Nebenprodukt, und zwar 1-(4-Hydroxyphenyl)-4-formyl-piperazin. Die Ethylacetat-Lösung (Filtrat) wurde eingedampft und der verbleibende Rückstand (12 g) an einer Kieselgel-$CH_2Cl_2$/Petrolether 1:2 Säule (ø 2,2 cm, H 44 cm) chromatographiert. Man eluierte mit $CH_2Cl_2$/Petrolether 1:2; 1:1 und 2:1 Mischungen, mit $CH_2Cl_2$ und mit $CH_2Cl_2/C_2H_5OH$-Mischungen (0,5-2,0 Vol.% $C_2H_5OH$). Nach Vorzonen wurden 2,82 g (= 22,7 % Ausbeute) 1-(4-Hydroxyphenyl)-4-(6-phenyl-pyrid-2-yl)-piperazin als hochviskoses Öl erhalten; Analyse: $C_{21}H_{21}N_3O$ (MG 331.42)
Ber. C 76.11, H 6.39, N 12.68;
Gef. C 75.7 , H 6.2 , N 12.3 %.

Beispiel 24

Nach der gleichen Arbeitsweise wie im Beispiel 23 beschrieben wurde im 25 mMol Maßstab ausgehend von 1-(4-Hydroxyphenyl)-piperazin-dihydrobromid, $K_2CO_3$ und 2-Chlor-6-cyclohexyl-4-methyl-pyridin (34 Stunden Rückflußkochen unter Rühren) in abs. DMF unter Zusatz von 5 mMol Natriumjodid 1-(4-Hydroxyphenyl)-4-(6-cyclohexyl-4-methyl-pyrid-2-yl)-piperazin, Fp. 82-83°C, in 15 %iger Ausbeute hergestellt; Analyse: $C_{22}H_{29}N_3O$ (MG 351.50)
Ber. C 75.18, H 8.32, N 11.96;
Gef. C 73.5 , H 8.5 , N 11.6 %.
Hauptprodukt ist unter diesen Reaktionsbedingungen 1-(4-Hydroxyphenyl)-4-formyl-piperazin, das in 74 %iger Ausbeute als unerwünschtes Produkt anfällt.

PATENTANSPRÜCHE:

1. Verbindung der Formel I

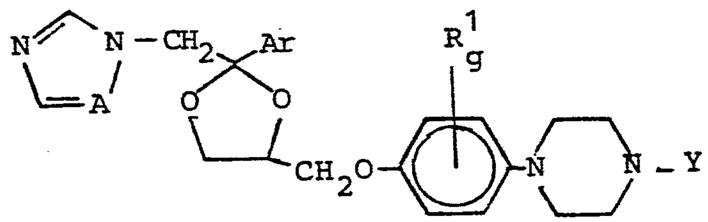

I,

in der bedeuten:

A CH oder N,

Ar Naphthyl, Thienyl, Halothienyl oder eine unsubstituierte oder eine bis zu 3 Substituenten tragende Phenylgruppe, wobei die Substituenten gleich oder verschieden sein können und Halogen, Trifluormethyl, $C_1$-$C_2$-Alkyl, $C_1$-$C_2$-Alkoxy oder Phenoxy bedeuten,

$R^1$ $C_1$-$C_3$-Alkyl, F oder Cl,

g 0, 1 oder 2,

Y die folgenden heterocyclischen Reste

a) 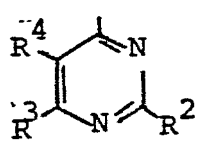 oder 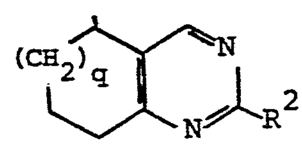 , in denen

$R^2$ $C_1$-$C_4$-Alkyl, eine unsubstituierte oder 1 oder 2 Substituenten tragende Phenylgruppe, wobei die Substituenten gleich oder verschieden sein können und Halogen, Trifluormethyl, Methoxy, Ethoxy, Nitro oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Methoxy-, Ethoxy- oder $C_1$-$C_3$-Alkyl bedeuten,

$R^3$ H, $C_1$-$C_8$-Alkyl, $C_3$-$C_6$-Cycloalkyl-$C_1$-$C_3$-alkyl, eine unsubstituierte oder 1 oder 2 Substituenten tragende Phenylgruppe, wobei die Substituenten gleich oder verschieden sein können und Halogen, Methoxy-, Ethoxy oder $C_1$-$C_3$-Alkyl, eine unsubstituierte oder im Phenylrest durch Methoxy, 1,2-Methylendioxy, F, Cl oder $C_1$-$C_3$-Alkyl substituierte Phenyl-$C_1$-$C_2$-alkylgruppe, oder Trifluormethyl bedeuten,

$R^4$ H, $C_1$-$C_4$-Alkyl oder Benzyl,
oder $R^3$ und $R^4$ zusammen
$-(CH_2)_r-$, wobei r=3 oder 4 ist, oder -CH=CH-CH=CH-,

q 0 oder 1 bedeutet, oder

b) 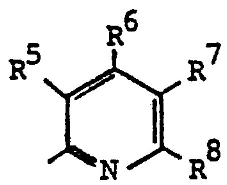 , in dem

$R^5$ H oder CN,

$R^6$ H, $C_1$-$C_4$-Alkyl oder eine unsubstituierte oder durch $OCH_3$, F, Cl, $CH_3$ oder $C_2H_5$ substituierte Phenylgruppe,

$R^7$ H, Benzyl, $CF_3$ oder $CH_3$,

$R^8$ $C_5$-$C_6$-Cycloalkyl oder eine unsubstituierte oder durch $OCH_3$, F, Cl, $CH_3$ oder $C_2H_5$ substituierte Phenylgruppe bedeutet und, falls $R^5$ CN und/oder $R^7$ $CF_3$ bedeutet, $R^8$ auch H bedeuten kann, oder

$R^7$ und $R^8$ zusammen $-(CH_2)_4-$ bedeuten, oder

c) 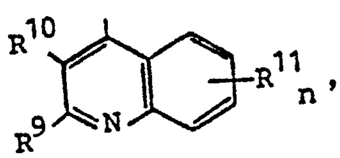 , in dem

$R^9$ H, Methyl oder Ethyl,

$R^{10}$ H, CN oder $COOR^{12}$, wobei $R^{12}$ Methyl oder Ethyl bedeutet,

$R^{11}$ $C_1$-$C_4$-Alkyl, $C_1$-$C_4$-Alkoxy, Halogen oder Trifluormethyl und

n 0, 1 oder 2 bedeute , wobei, falls $R^{11}$ $CF_3$ bedeutet, n=1 ist, und, falls n≠0, die Reste $R^{11}$ in 5-, 6-, 7- oder 8-Stellung des Chinolinsystems stehen können, oder

d) 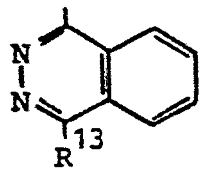 , in dem

$R^{13}$ H, $C_1$-$C_4$-Alkyl oder eine unsubstituierte oder 1 oder 2 Substituenten tragende Phenylgruppe, wobei die Substituenten gleich oder verschieden sein können und Halogen, Methoxy, Ethoxy, Methyl oder Ethyl bedeuten, bedeutet,

sowie deren physiologisch verträgliche Säureadditionssalze.

2. Verbindung I nach Anspruch 1, dadurch gekennzeichnet, daß mindestens einer der Substituenten die folgende Bedeutung hat:

A CH oder N,

Ar eine durch 1 oder 2 F- oder Cl-Atome substituierte Phenylgruppe,

$R^1$ $CH_3$ oder $C_2H_5$,

g 0 oder 2,

Y

zu dem heterocyclischen Rest a)

$R^2$ $C_1$-$C_4$-Alkyl, eine unsubstituierte oder 1 oder 2 gleiche oder verschiedene Substituenten tragende Phenylgruppe, wobei die Substituenten F, Cl, $OCH_3$, $OC_2H_5$, $CH_3$ oder $C_2H_5$ bedeuten, oder eine Benzyl- oder eine durch ein F- oder Cl-Atom im Phenylrest substituierte Benzylgruppe,

$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert, oder $CF_3$,

$R^4$ $C_1$-$C_4$-Alkyl, Benzyl oder

$R^3$ und $R^4$ zusammen $-(CH_2)_3-$, $-(CH_2)_4-$ oder -CH=CH-CH=CH-,

q 0 oder 1,

zu dem heterocyclischen Rest b)

$R^5$ H oder CN,

$R^6$ H, $CH_3$ oder Phenyl,

$R^7$ H oder $CF_3$,

$R^8$ Phenyl oder durch F, Cl, $CH_3$ oder $OCH_3$ substituiertes Phenyl oder, falls $R^5$ CN und/oder $R^7$ $CF_3$ bedeutet, zusätzlich H und

$R^7$ und $R^8$ zusammen $-(CH_2)_4-$,

zu dem heterocyclischen Rest c)

$R^9$ H

$R^{10}$ CN, $COOCH_3$ oder $COOC_2H_5$,

$R^{11}$ $C_1$-$C_4$-Alkyl, $C_1$-$C_4$-Alkoxy, F, Cl, Br oder $CF_3$,

n 0, 1 oder 2, wobei falls $R^{11}$ $CF_3$ bedeutet, n=1 ist und, falls n ungleich 0 ist, $R^{11}$ in 5, 6, 7 oder 8-Stellung stehen kann,

zu dem heterocyclischen Rest d)

$R^{13}$ H, $C_1$-$C_4$-Alkyl oder eine unsubstituierte oder durch 1 oder 2 F, Cl, $OCH_3$ oder $CH_3$ substituierte Phenylgruppe.

3. Verbindung I nach Anspruch 1, dadurch gekennzeichnet, daß mindestens einer der Substituenten bzw. Indizes die folgende Bedeutung hat:

A CH oder N,

Ar 2,4-Dichlorphenyl,

$R^1$ $CH_3$,

g 0 oder 2,

Y

zu den heterocyclischen Resten a)

$R^2$ eine unsubstituierte oder 1 oder 2 gleiche oder verschiedene Substituenten tragende Phenylgruppe, wobei die Substituenten Cl, $OCH_3$, $OC_2H_5$ oder $CH_3$ bedeuten, eine Benzyl- oder eine Chlorbenzylgruppe,

$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert,

$R^4$ $C_1$-$C_4$-Alkyl, Benzyl oder

$R^3$ und $R^4$ zusammen $-(CH_2)_3-$, $-(CH_2)_4-$ oder -CH=CH-CH=CH-,

q 0 oder 1,

zu dem heterocyclischen Rest b)

$R^5$ H oder CN,

$R^6$ H oder $CH_3$,

$R^7$ H oder $CF_3$,

$R^8$ Phenyl oder durch Cl oder $OCH_3$ substituiertes Phenyl oder, falls $R^5$ CN und/oder $R^7$ $CF_3$ bedeutet, zusätzlich H,

zu dem heterocyclischen Rest c)

$R^9$ H,

$R^{10}$ CN, $COOCH_3$ oder $COOC_2H_5$,

$R^{11}$ $CH_3$, $C_2H_5$, $OCH_3$, $OC_2H_5$, F, Cl, Br oder $CF_3$,

n 0, 1 oder 2, wobei, falls $R^{11}$ $CF_3$ bedeutet, n=1 ist und, falls n ungleich 0 ist, $R^{11}$ in 5-, 6-, 7- oder 8-Stellung stehen kann,

zu dem heterocyclischen Rest d)

$R^{13}$ eine unsubstituierte oder durch 1 oder 2 F, Cl, $OCH_3$ oder $CH_3$ substituierte Phenylgruppe.

4. Verfahren zum Herstellen einer Verbindung I nach Anspruch 1, dadurch gekennzeichnet, daß man

A) eine Verbindung der Formel II,

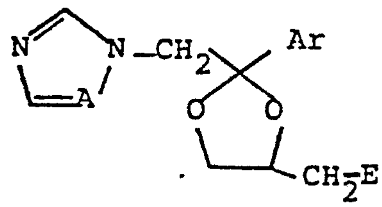

II

in der
A und Ar die zu Formel I angegebenen Bedeutungen haben und
E Halogen oder Acyloxy, Alkyl-sulfonyloxy oder Arylsulfonyloxy bedeutet,
mit einer Verbindung der Formel III

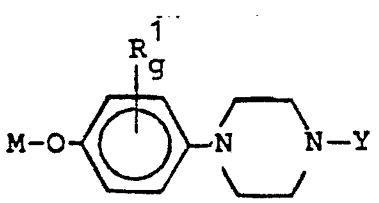

III,

in der
M H, ein Alkali- oder Erdalkalimetall bedeutet und
$R^1$, g und Y die zu Formel I angegebenen Bedeutungen haben, umsetzt, oder daß man

B) eine Verbindung der Formel IV,

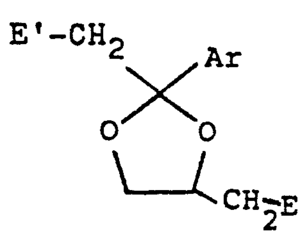

IV

in der

Ar die zu Formel I und E und E' die zu Formel II für E angegebenen Bedeutungen haben,
zunächst mit einer Verbindung der Formel III umsetzt und hierbei eine Verbindung der Formel V herstellt,

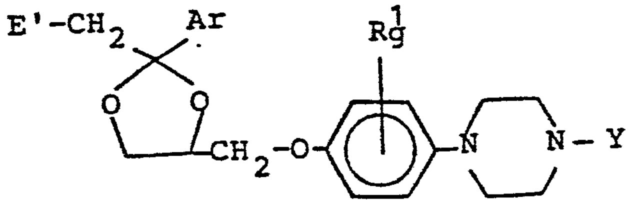

V

in der
Ar, $R^1$, g und Y die zu Formel I und E' die zu Formel II für E angegebenen Bedeutungen haben,
und anschließend eine Verbindung der Formel V mit einer Verbindung der Formel VI umsetzt,

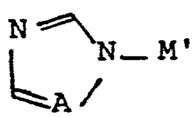

VI

in der A CH oder N und
M' H, ein Alkali- oder Erdalkalimetall oder $Si(CH_3)_3$ bedeutet,
oder daß man
C) eine Verbindung der Formel VII,

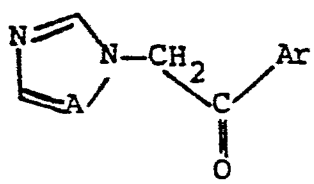

VII

in der A und Ar die zu Formel I angegebenen Bedeutungen haben, mit einem 1,2-Diol der Formel VIII,

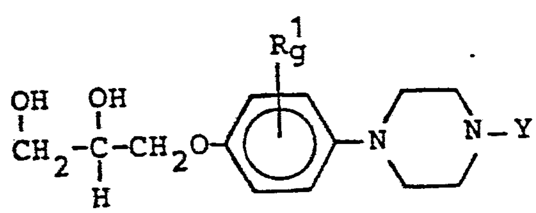

VIII

in der $R^1$, g und Y die zu Formel I angegebenen Bedeutungen haben, umsetzt,

oder daß man
D) eine Verbindung der Formel IX,

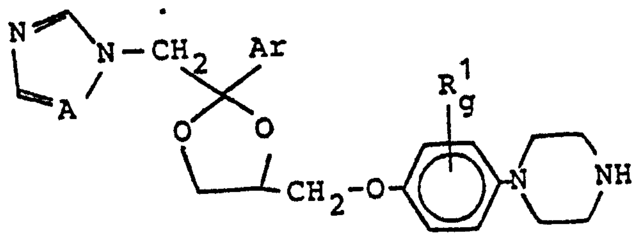

IX

in der A, Ar, $R^1$ und g die zu Formel I angegebenen Bedeutungen haben, mit einer Verbindung der Formel X,

E"-Y                                                    X

in der E" $C_1$-$C_4$-Alkoxy, Cl, Br, J, Acyloxy, Alkylsulfonyloxy oder Arylsulfonyloxy bedeutet und Y die zu Formel I unter a, b, c und d angegebenen Bedeutungen hat, umsetzt, und gegebenenfalls die nach Weg A)-D) erhaltenen Verbindungen der Formel I mit anorganischen oder organischen Säuren in ihre physiologisch verträglichen Säureadditionssalze überführt.

5. Verbindung der Formel IIIa,

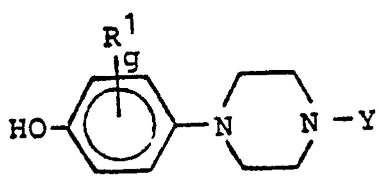

IIIa

in der bedeuten:

$R^1$   $C_1$-$C_3$-Alkyl, F oder Cl,

g   0, 1 oder 2 und

Y   die folgenden heterocyclischen Reste

a) 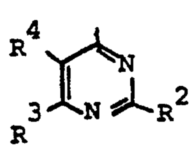 oder 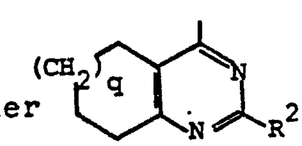 , in denen

$R^2$ $C_1$-$C_4$-Alkyl, eine unsubstituierte oder 1 oder 2 Substituenten tragende Phenylgruppe, wobei die Substituenten gleich oder verschieden sein können und Halogen, Trifluormethyl, Methoxy, Ethoxy, Nitro oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Methoxy-, Ethoxy- oder $C_1$-$C_3$-Alkyl bedeuten,

$R^3$ H, $C_1$-$C_8$-Alkyl, $C_3$-$C_6$-Cycloalkyl-$C_1$-$C_3$-alkyl, eine unsubstituierte oder 1 oder 2 Substituenten tragende Phenylgruppe, wobei die Substituenten gleich oder verschieden sein können und Halogen, Methoxy-, Ethoxy oder $C_1$-$C_3$-Alkyl, eine unsubstituierte oder im Phenylrest durch Methoxy, 1,2-Methylendioxy, F, Cl oder $C_1$-$C_3$-Alkyl substituierte Phenyl-$C_1$-$C_2$-alkylgruppe, oder Trifluormethyl bedeuten,

$R^4$ H, $C_1$-$C_4$-Alkyl oder Benzyl,
oder $R^3$ und $R^4$ zusammen
$-(CH_2)_r-$, wobei r=3 oder 4 ist, oder -CH=CH-CH=CH-,

q O oder 1 bedeutet, oder

b) 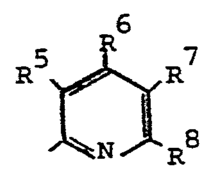 , in dem

$R^5$ H oder CN,

$R^6$ H, $C_1$-$C_4$-Alkyl oder eine unsubstituierte oder durch $OCH_3$, F, Cl, $CH_3$ oder $C_2H_5$ substituierte Phenylgruppe,

$R^7$ H, Benzyl oder $CF_3$,

$R^8$ $C_5$-$C_6$-Cycloalkyl oder eine unsubstituierte oder durch $OCH_3$, F, Cl, $CH_3$ oder $C_2H_5$ substituierte Phenylgruppe bedeuten und, falls $R^5$ CN und/oder $R^7$ $CF_3$ bedeutet,

$R^8$ auch H bedeuten kann, oder

$R^7$ und $R^8$ zusammen $-(CH_2)_4-$ bedeuten, oder

c) 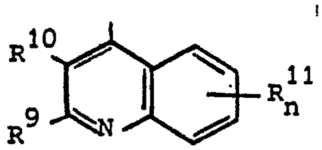, in dem

$R^9$ H, Methyl oder Ethyl,

$R^{10}$ H, CN oder $COOR^{12}$, wobei $R^{12}$ Methyl oder Ethyl bedeutet,

$R^{11}$ $C_1$-$C_4$-Alkyl, $C_1$-$C_4$-Alkoxy, Halogen oder Trifluormethyl und

n 0, 1 oder 2 bedeuten, wobei, falls $R^{11}$ $CF_3$ bedeutet, n=1 ist, und, falls n ungleich 0, die Reste $R^{11}$ in 5-, 6-, 7- oder 8-Stellung des Chinolinsystems stehen können, oder

d) 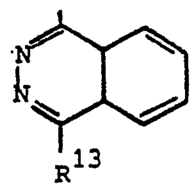, in dem

$R^{13}$ H, $C_1$-$C_4$-Alkyl oder eine unsubstituierte oder 1 oder 2 Substituenten tragende Phenylgruppe, wobei die Substituenten gleich oder verschieden sein können und Halogen, Methoxy, Ethoxy, Methyl oder Ethyl bedeuten,

sowie deren Säureadditionssalze.

6. Verfahren zum Herstellen einer Verbindung IIIa nach Anspruch 5, dadurch gekennzeichnet, daß man eine Verbindung der Formel XIII,

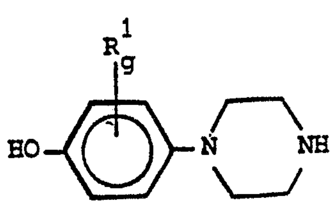

XIII,

in der $R^1$ und g die zu Formel I angegebenen Bedeutungen haben, oder ein Salz dieser Verbindung, mit einer Verbindung der Formel X,

E"-Y X,

in der E" $C_1$-$C_4$-Alkoxy, Cl, Br, J, Acyloxy, Alkylsulfonyloxy oder Arylsulfonyloxy bedeutet, und

Y die zur Formel I angegebenen Bedeutungen hat,

umsetzt und gegebenenfalls die erhaltenen Verbindungen der Formel IIIa mit anorganischen oder organischen Säuren in ihre Säureadditionssalze überführt.

7. Verwendung einer Verbindung der Formel I nach Anspruch 1 als Antimykotikum.

8. Verwendung einer Verbindung der Formel I nach Anspruch 1 zur Herstellung eines antimykotisch wirkenden Arzneimittels.

9. Arzneimittel mit antimykotischer Wirkung, gekennzeichnet durch einen wirksamen Gehalt einer Verbindung I nach Anspruch 1.

10. Verfahren zum Behandeln von Mykosen, dadurch gekennzeichnet, daß man eine wirksame Menge einer Verbindung I nach Anspruch 1 zusammen mit pharmazeutisch geeigneten Trägerstoffen appliziert.

11. Verbindung I nach Anspruch 1, dadurch gekennzeichnet, daß der Azolylmethylrest und die 4-ständige Piperazinophenoxymethylgruppe am Dioxolanring in cis-Stellung stehen.

Patentansprüche Griechenland, Spanien und Österreich:

1. Verfahren zum Herstellen einer Verbindung I

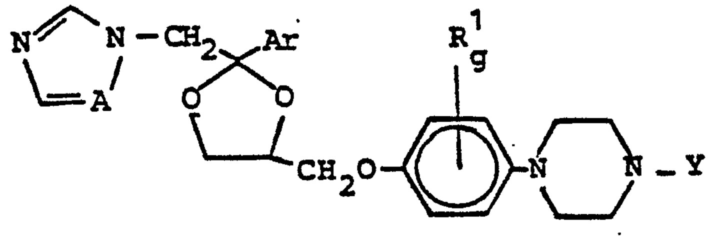

I,

in der bedeuten:

A CH oder N,

Ar Naphthyl, Thienyl, Halothienyl oder eine unsubstituierte oder eine bis zu 3 Substituenten tragende Phenylgruppe, wobei die Substituenten gleich oder verschieden sein können und Halogen, Trifluormethyl, $C_1$-$C_2$-Alkyl, $C_1$-$C_2$-Alkoxy oder Phenoxy bedeuten,

$R^1$ $C_1$-$C_3$-Alkyl, F oder Cl,

g 0, 1 oder 2,

Y die folgenden heterocyclischen Reste

a) 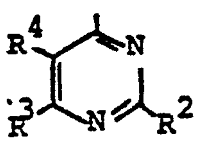 oder 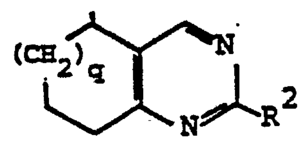 , in denen

$R^2$ $C_1$-$C_4$-Alkyl, eine unsubstituierte oder 1 oder 2 Substituenten tragende Phenylgruppe, wobei die Substituenten gleich oder verschieden sein können und Halogen, Trifluormethyl, Methoxy, Ethoxy, Nitro oder $C_1$-$C_4$-Alkyl bedeuten,
oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Methoxy-, Ethoxy- oder $C_1$-$C_3$-Alkyl bedeuten,

$R^3$ H, $C_1$-$C_8$-Alkyl, $C_3$-$C_6$-Cycloalkyl-$C_1$-$C_3$-alkyl, eine unsubstituierte oder 1 oder 2 Substituenten tragende Phenylgruppe, wobei die Substituenten gleich oder verschieden sein können und Halogen, Methoxy-, Ethoxy oder $C_1$-$C_3$-Alkyl, eine unsubstituierte oder im Phenylrest durch Methoxy, 1,2-Methylendioxy, F, Cl oder $C_1$-$C_3$-Alkyl substituierte Phenyl-$C_1$-$C_2$-alkylgruppe, oder Trifluormethyl bedeuten,

$R^4$ H, $C_1$-$C_4$-Alkyl oder Benzyl,
oder $R^3$ und $R^4$ zusammen
$-(CH_2)_r-$, wobei r=3 oder 4 ist, oder -CH=CH-CH=CH-,
q 0 oder 1 bedeutet, oder

b) 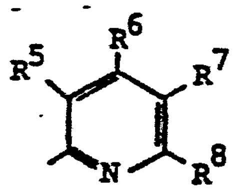 , in dem

$R^5$ H oder CN,
$R^6$ H, $C_1$-$C_4$-Alkyl oder eine unsubstituierte oder durch $OCH_3$, F, Cl, $CH_3$ oder $C_2H_5$ substituierte Phenylgruppe,
$R^7$ H, Benzyl, $CF_3$ oder $CH_3$,
$R^8$ $C_5$-$C_6$-Cycloalkyl oder eine unsubstituierte oder durch $OCH_3$, F, Cl, $CH_3$ oder $C_2H_5$ substituierte Phenylgruppe bedeutet und, falls $R^5$ CN und/oder $R^7$ $CF_3$ bedeutet, $R^8$ auch H bedeuten kann, oder
$R^7$ und $R^8$ zusammen $-(CH_2)_4-$ bedeuten, oder

c) 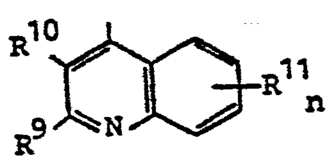, in dem

$R^9$ H, Methyl oder Ethyl,
$R^{10}$ H, CN oder $COOR^{12}$, wobei $R^{12}$ Methyl oder Ethyl bedeutet,

$R^{11}$ $C_1$-$C_4$-Alkyl, $C_1$-$C_4$-Alkoxy, Halogen oder Trifluormethyl und

n 0, 1 oder 2 bedeute , wobei, falls $R^{11}$ $CF_3$ bedeutet, n=1 ist, und, falls n≠0, die Reste $R^{11}$ in 5-, 6-, 7- oder 8-Stellung des Chinolinsystems stehen können, oder

d) 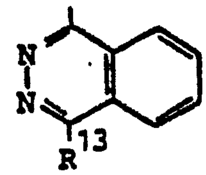 , in dem

$R^{13}$ H, $C_1$-$C_4$-Alkyl oder eine unsubstituierte oder 1 oder 2 Substituenten tragende Phenylgruppe, wobei die Substituenten gleich oder verschieden sein können und Halogen, Methoxy, Ethoxy, Methyl oder Ethyl bedeuten, bedeutet,

sowie von deren physiologisch verträglichen Säureadditionssalzen, dadurch gekennzeichnet, daß man

A) eine Verbindung der Formel II,

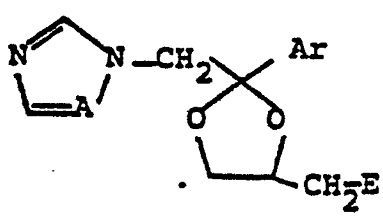

II

in der

A und Ar die zu Formel I angegebenen Bedeutungen haben und

E Halogen oder Acyloxy, Alkyl-sulfonyloxy oder Arylsulfonyloxy bedeutet,

mit einer Verbindung der Formel III

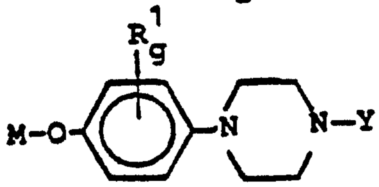

III,

in der

M H, ein Alkali- oder Erdalkalimetall bedeutet und

$R^1$, g und Y die zu Formel I angegebenen Bedeutungen haben, umsetzt, oder daß man

B) eine Verbindung der Formel IV,

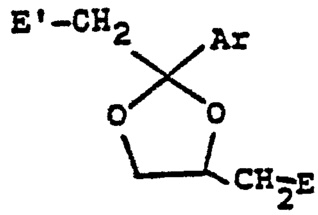

IV

in der

Ar die zu Formel I und E und E' die zu Formel II für E angegebenen Bedeutungen haben,
zunächst mit einer Verbindung der Formel III umsetzt und hierbei eine Verbindung der Formel V herstellt,

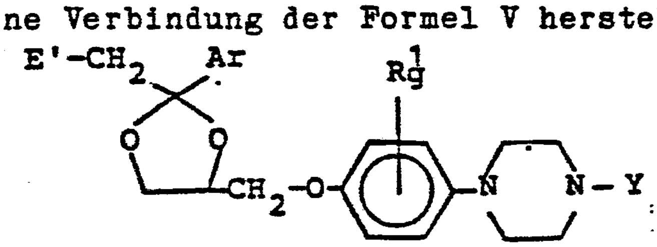

V

in der
Ar, $R^1$, g und Y die zu Formel I und E' die zu Formel II für E angegebenen Bedeutungen haben,
und anschließend eine Verbindung der Formel V mit einer Verbindung der Formel VI umsetzt,

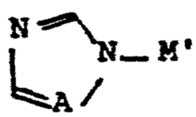

VI

in der A CH oder N und
M' H, ein Alkali- oder Erdalkalimetall oder $Si(CH_3)_3$ bedeutet,
oder daß man
C) eine Verbindung der Formel VII,

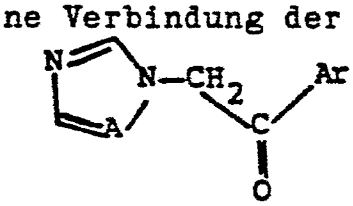

VII

in der A und Ar die zu Formel I angegebenen Bedeutungen haben, mit einem 1,2-Diol der Formel VIII,

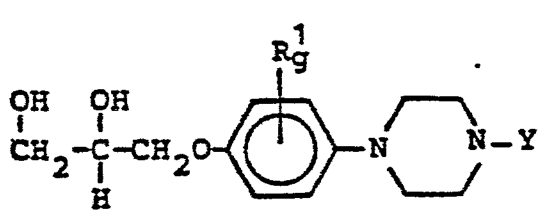

VIII

in der $R^1$, g und Y die zu Formel I angegebenen Bedeutungen haben, umsetzt,

oder daß man

D) eine Verbindung der Formel IX,

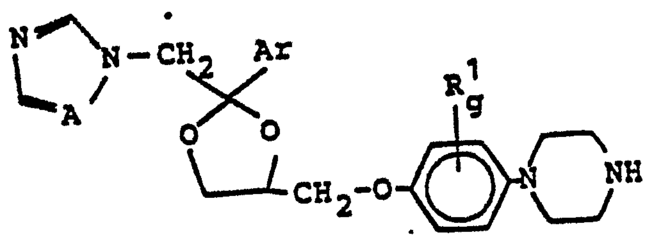

IX

in der A, Ar, $R^1$ und g die zu Formel I angegebenen Bedeutungen haben, mit einer Verbindung der Formel X,

E"-Y X

in der E" $C_1$-$C_4$-Alkoxy, Cl, Br, J, Acyloxy, Alkylsulfonyloxy oder Arylsulfonyloxy bedeutet und Y die zu Formel I unter a, b, c und d angegebenen Bedeutungen hat, umsetzt, und gegebenenfalls die nach Weg A)-D) erhaltenen Verbindungen der Formel I mit anorganischen oder organischen Säuren in ihre physiologisch verträglichen Säureadditionssalze überführt.

2. Verfahren zum Herstellen einer Verbindung I nach Anspruch 1, dadurch gekennzeichnet, daß mindestens einer der Substituenten die folgende Bedeutung hat:

A CH oder N,

Ar eine durch 1 oder 2 F- oder Cl-Atome substituierte Phenylgruppe,

$R^1$ $CH_3$ oder $C_2H_5$,

g 0 oder 2,

Y

zu dem heterocyclischen Rest a)

$R^2$ $C_1$-$C_4$-Alkyl, eine unsubstituierte oder 1 oder 2 gleiche oder verschiedene Substituenten tragende Phenylgruppe, wobei die Substituenten F, Cl, $OCH_3$, $OC_2H_5$,

$CH_3$ oder $C_2H_5$ bedeuten, oder eine Benzyl- oder eine durch ein F- oder Cl-Atom im Phenylrest substituierte Benzylgruppe,

$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert, oder $CF_3$,

$R^4$ $C_1$-$C_4$-Alkyl, Benzyl oder

$R^3$ und $R^4$ zusammen $-(CH_2)_3-$, $-(CH_2)_4-$ oder -CH=CH-CH=CH-,

q 0 oder 1,

zu dem heterocyclischen Rest b)

$R^5$ H oder CN,

$R^6$ H, $CH_3$ oder Phenyl,

$R^7$ H oder $CF_3$,

$R^8$ Phenyl oder durch F, Cl, $CH_3$ oder $OCH_3$ substituiertes Phenyl oder, falls $R^5$ CN und/oder $R^7$ $CF_3$ bedeutet, zusätzlich H und

$R^7$ und $R^8$ zusammen $-(CH_2)_4-$,

zu dem heterocyclischen Rest c)

$R^9$ H

$R^{10}$ CN, $COOCH_3$ oder $COOC_2H_5$,

$R^{11}$ $C_1$-$C_4$-Alkyl, $C_1$-$C_4$-Alkoxy, F, Cl, Br oder $CF_3$,

n 0, 1 oder 2, wobei falls $R^{11}$ $CF_3$ bedeutet, n=1 ist und, falls n ungleich 0 ist, $R^{11}$ in 5, 6, 7 oder 8-Stellung stehen kann,

zu dem heterocyclischen Rest d)

$R^{13}$ H, $C_1$-$C_4$-Alkyl oder eine unsubstituierte oder durch 1 oder 2 F, Cl, $OCH_3$ oder $CH_3$ substituierte Phenylgruppe.

3. Verfahren zum Herstellen einer Verbindung I nach Anspruch 1, dadurch gekennzeichnet, daß mindestens einer der Substituenten bzw. Indizes die folgende Bedeutung hat:

A CH oder N,

Ar 2,4-Dichlorphenyl,

$R^1$ $CH_3$,

g 0 oder 2,

Y

zu den heterocyclischen Resten a)

$R^2$ eine unsubstituierte oder 1 oder 2 gleiche oder verschiedene Substituenten tragende Phenylgruppe, wobei die Substituenten Cl, $OCH_3$, $OC_2H_5$ oder $CH_3$ bedeuten, eine Benzyl- oder eine Chlorbenzylgruppe,

$R^3$ $C_1$-$C_8$-Alkyl, $C_5$-$C_6$-Cycloalkyl-$C_1$-$C_2$-alkyl, eine Phenyl- oder Phenyl-$C_1$-$C_2$-alkylgruppe, im Phenylrest jeweils unsubstituiert oder durch 1 oder 2 F, Cl, $OCH_3$ oder $CH_3$ substituiert,

$R^4$ $C_1$-$C_4$-Alkyl, Benzyl oder

$R^3$ und $R^4$ zusammen $-(CH_2)_3-$, $-(CH_2)_4-$ oder -CH=CH-CH=CH-,

q 0 oder 1,

zu dem heterocyclischen Rest b)

$R^5$ H oder CN,

$R^6$ H oder $CH_3$,

$R^7$ H oder $CF_3$,

$R^8$ Phenyl oder durch Cl oder $OCH_3$ substituiertes Phenyl oder, falls $R^5$ CN und/oder $R^7$ $CF_3$ bedeutet, zusätzlich H,

zu dem heterocyclischen Rest c)

$R^9$ H,

$R^{10}$ CN, $COOCH_3$ oder $COOC_2H_5$,

$R^{11}$ $CH_3$, $C_2H_5$, $OCH_3$, $OC_2H_5$, F, Cl, Br oder $CF_3$,

n 0, 1 oder 2, wobei, falls $R^{11}$ $CF_3$ bedeutet, n=1 ist und, falls n ungleich 0 ist, $R^{11}$ in 5-, 6-, 7- oder 8-Stellung stehen kann,

zu dem heterocyclischen Rest d)

$R^{13}$ eine unsubstituierte oder durch 1 oder 2 F, Cl, $OCH_3$ oder $CH_3$ substituierte Phenylgruppe.

4. Verfahren zum Herstellen einer Verbindung IIIa

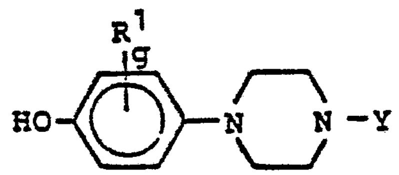

IIIa

in der bedeuten:

$R^1$ $C_1$-$C_3$-Alkyl, F oder Cl,

g 0, 1 oder 2 und

Y die folgenden heterocyclischen Reste

a) 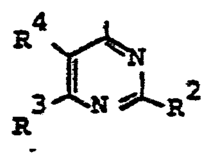 oder 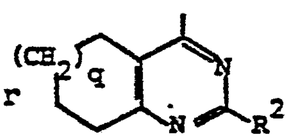 , in denen

$R^2$ $C_1$-$C_4$-Alkyl, eine unsubstituierte oder 1 oder 2 Substituenten tragende Phenylgruppe, wobei die Substituenten gleich oder verschieden sein können und Halogen, Trifluormethyl, Methoxy, Ethoxy, Nitro oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Methoxy-, Ethoxy- oder $C_1$-$C_3$-Alkyl bedeuten,

$R^3$ H, $C_1$-$C_8$-Alkyl, $C_3$-$C_6$-Cycloalkyl-$C_1$-$C_3$-alkyl, eine unsubstituierte oder 1 oder 2 Substituenten tragende Phenylgruppe, wobei die Substituenten gleich oder verschieden sein können und Halogen, Methoxy-, Ethoxy oder $C_1$-$C_3$-Alkyl, eine unsubstituierte oder im Phenylrest durch Methoxy, 1,2-Methylendioxy, F, Cl oder $C_1$-$C_3$-Alkyl substituierte Phenyl-$C_1$-$C_2$-alkylgruppe, oder Trifluormethyl bedeuten,

$R^4$ H, $C_1$-$C_4$-Alkyl oder Benzyl, oder $R^3$ und $R^4$ zusammen $-(CH_2)_r-$, wobei r=3 oder 4 ist, oder -CH=CH-CH=CH-,

q 0 oder 1 bedeutet, oder

b) 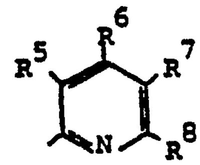 , in dem

$R^5$ H oder CN,

$R^6$ H, $C_1$-$C_4$-Alkyl oder eine unsubstituierte oder durch $OCH_3$, F, Cl, $CH_3$ oder $C_2H_5$ substituierte Phenylgruppe,

$R^7$ H, Benzyl oder $CF_3$,

$R^8$ $C_5$-$C_6$-Cycloalkyl oder eine unsubstituierte oder durch $OCH_3$, F, Cl, $CH_3$ oder $C_2H_5$ substituierte Phenylgruppe bedeuten und, falls $R^5$ CN und/oder $R^7$ $CF_3$ bedeutet,

$R^8$ auch H bedeuten kann, oder

$R^7$ und $R^8$ zusammen $-(CH_2)_4-$ bedeuten, oder

c) 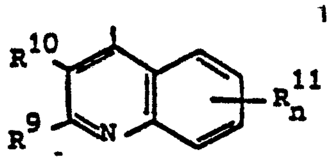 , in dem

$R^9$ H, Methyl oder Ethyl,

$R^{10}$ H, CN oder $COOR^{12}$, wobei $R^{12}$ Methyl oder Ethyl bedeutet,

$R^{11}$ $C_1$-$C_4$-Alkyl, $C_1$-$C_4$-Alkoxy, Halogen oder Trifluormethyl und

n 0, 1 oder 2 bedeuten, wobei, falls $R^{11}$ $CF_3$ bedeutet, n=1 ist, und, falls n ungleich 0, die Reste $R^{11}$ in 5-, 6-, 7- oder 8-Stellung des Chinolinsystems stehen können, oder

d) 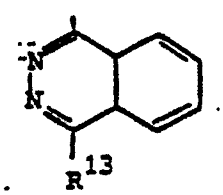 , in dem

$R^{13}$ H, $C_1$-$C_4$-Alkyl oder eine unsubstituierte oder 1 oder 2 Substituenten tragende Phenylgruppe, wobei die Substituenten gleich oder verschieden sein

können und Halogen, Methoxy, Ethoxy, Methyl oder Ethyl bedeuten,

sowie von deren Säureadditionssalzen,
dadurch gekennzeichnet, daß man eine Verbindung der Formel XIII,

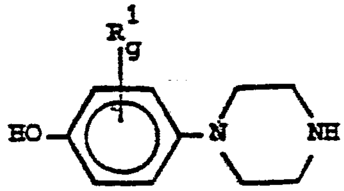

XIII,

in der $R^1$ und g die zu Formel I angegebenen Bedeutungen haben, oder ein Salz dieser Verbindung, mit einer Verbindung der Formel X,

$$E''-Y \qquad X,$$

in der E" $C_1$-$C_4$-Alkoxy, Cl, Br, J, Acyloxy, Alkylsulfonyloxy oder Arylsulfonyloxy bedeutet, und

Y die zur Formel I angegebenen Bedeutungen hat,

umsetzt und gegebenenfalls die erhaltenen Verbindungen der Formel IIIa mit anorganischen oder organischen Säuren in ihre Säureadditionssalze überführt.

5. Verwendung einer Verbindung der Formel I nach Anspruch 1 als Antimykotikum.

6. Verwendung einer Verbindung der Formel I nach Anspruch 1 zur Herstellung eines antimykotisch wirkenden Arzneimittels.